(19) Europäisches Patentamt / European Patent Office / Office européen des brevets

(11) **EP 4 327 855 A2**

(12) **EUROPEAN PATENT APPLICATION**

(43) Date of publication:
**28.02.2024 Bulletin 2024/09**

(21) Application number: **23199642.2**

(22) Date of filing: **21.11.2019**

(51) International Patent Classification (IPC):
***A61M 27/00*** *(2006.01)*

(52) Cooperative Patent Classification (CPC):
**A61M 27/00; A61M 25/0017; A61M 25/007**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.11.2018 DK PA201800892**

(62) Document number(s) of the earlier application(s) in accordance with Art. 76 EPC:
**19809378.3 / 3 883 630**

(71) Applicant: **Coloplast A/S**
**3050 Humlebaek (DK)**

(72) Inventors:
• **Oeelund, Jakob**
 **3450 Alleroed (DK)**

• **Hede, Anders Wilhelm**
 **2900 Hellerup (DK)**
• **Soerensen, Caecilie**
 **2750 Ballerup (DK)**
• **Moelleskov, Lasse Hylleberg**
 **DK-2200 Copenhagen N (DK)**
• **Pedersen, Troels Gottfried**
 **2990 Nivaa (DK)**

Remarks:
 This application was filed on 26-09-2023 as a divisional application to the application mentioned under INID code 62.

(54) **AN INTERMITTENT URINARY CATHETER**

(57) An intermittent urinary catheter is provided. The intermittent urinary catheter includes multiple drainage openings providing for inflow or urine into the catheter. The drainage openings have $\alpha$ largest dimension below 1 mm. With this catheter, the bladder wall is less exposed to large negative suction during catheterisation, thus reducing the risk of influencing the bladder wall. Smaller drainage openings provide $\alpha$ reduced risk of influence to the urethra as well.

EP 4 327 855 A2

**Description**

The invention relates to an intermittent urinary catheter, methods and use of an intermittent urinary catheter.

**Brief Description of the Drawing**

The accompanying drawings are included to provide a further understanding of embodiments and are incorporated into and a part of this specification. The drawings illustrate embodiments and together with the description serve to explain principles of embodiments. Other embodiments and many of the intended advantages of embodiments will be readily appreciated as they become better understood by reference to the following detailed description. The elements of the drawings are not necessarily to scale relative to each other. Like reference numerals designate corresponding similar parts.

Figures 1 to 4 illustrate problems, which may occur with intermittent urinary catheters of the prior art.

Figure 5 illustrates one embodiment of an intermittent urinary catheter having a plurality of small drainage openings; the catheter is illustrated in a projective view.

Figure 6, 7, 8, 9A and 9B illustrate the function of embodiments of an intermittent urinary catheter.

Figures 10 to 17 illustrates various embodiments of an intermittent urinary catheter.

Figures 18 to 20 illustrate the magnitude of pressure pulses in an intermittent urinary catheter.

Figures 21A, 21B, 22 and 23 illustrates the pressure pulse as a function of the size of the drainage openings.

Figures 24 to 26 illustrate test set-ups used to determine a pressure pulse in an intermittent urinary catheter.

Figures 28 to 36 illustrate test results from testing of intermittent urinary catheters in a bladder-model.

Figures 37A, 37B and 38, 39 illustrate the distribution of inflow in a prior art catheter and in a catheter according to embodiments of this disclosure. Figures 37A, 37b are schematic illustrations and Figures 38 and 39 illustrate simulations.

Figure 40 illustrates how the inflow into a drainage opening may attract the bladder wall tissue.

Figures 41 and 42 is a schematic illustration of the distribution of flow over an external surface of a catheter, Figure 41 is a catheter according to embodiments of this disclosure and figure 42 is a prior art catheter.

Figures 43 to 61 illustrate simulations of flow and pressure in prior art catheters as well as in catheters according to this disclosure.

Figures 62 and 63 illustrate schematic views of test set-ups used for testing drag-force and pressure in the vicinity of a catheter during draining.

Figure 64 illustrate a schematic view of the distribution of pressure in a prior art catheter.

Figures 65 to 69 illustrate simulations of pressure in prior art catheters.

Figure 70 illustrate a schematic view of the distribution of pressure in a catheter according to embodiments of this disclosure.

Figures 71 to 73 illustrate simulations of pressure in a catheter according to embodiments of this disclosure.

Figure 74 illustrate a schematic view of the distribution of pressure in a catheter according to embodiments of this disclosure.

Figures 75 to 77 illustrate simulations of pressure in a catheter according to embodiments of this disclosure.

## Detailed Description

Embodiments relate to an intermittent urinary catheter having a tip portion with a tip in a proximal insertion end of the catheter, a tubular portion extending from the tip portion to a distal outlet end; the tubular portion having an inside lumen and a drainage portion provided with drainage openings configured for allowing urine to enter the inside lumen, the drainage portion being provided with more than 12 drainage openings.

An intermittent urinary catheter having a tip portion with a tip in a proximal insertion end of the catheter, a tubular portion extending from the tip portion to a distal outlet end; the tubular portion having an inside lumen and a drainage portion provided with drainage openings configured for allowing urine to enter the inside lumen, the drainage portion being provided with drainage openings each having a cross-sectional area of less than 0.4 mm$^2$.

Embodiments of this disclosure has the effect of providing an intermittent urinary catheter with significantly reduced risk of influencing the bladder wall and ureteral tissue during intermittent catheterisation. Furthermore, the catheterization procedure of emptying the bladder will be easier, not requiring re-positioning of the catheter, thus leading to a higher probability of emptying the bladder to a satisfactory level at every catherization.

During intermittent catheterisation and emptying of the bladder, the bladder contracts and eventually the bladder wall will get close to the catheter. The pressure differential between the bladder and the external surroundings creates an outflow of urine from the bladder through the catheter. If all drainage openings in the intermittent urinary catheter are suddenly blocked by bladder wall tissue a negative pressure pulse arises in the catheter due to the moving water column of urine in the catheter abruptly being stopped. This negative pressure provides a sudden suction of tissue towards the drainage openings and, if the negative pressure is maintained, maybe even into the inner lumen of the catheter. This phenomenon will in the context of this disclosure be referred to as clogging. The suction may influence the bladder wall tissue. The magnitude of the negative pressure is dependent inter alia on the abruptness of the blockage of the drainage openings and the flow rate. If the catheter is a prior art intermittent catheter, such as one commonly provided with two drainage openings, one of the drainage openings may be clogged by bladder wall tissue which may result only in a limited negative pressure pulse, but if/when the second and last drainage opening is also clogged by bladder wall tissue, the urine flow through the catheter is abruptly stopped, resulting in a significant negative pressure pulse in the catheter. This causes the tissue in proximity of the drainage openings to be sucked into the lumen of the catheter through the drainage openings. The occurrence of this negative pressure pulse sucking the bladder tissue into the drainage openings may be what some catheter users sense as a pinch in the bladder.

Contrary to these drawbacks of commonly available catheters, the present disclosure provides an intermittent urinary catheter which uses multiple drainage openings that hinder the possibility of an abrupt closure of all drainage openings almost simultaneously, thereby eradicating the occurrence of a negative pressure pulse sucking bladder wall tissue towards and into the drainage openings. The multiple drainage openings described herein secures that during voiding, when contact between the bladder wall and catheter occurs, potential blockage of the drainage openings only takes place gradually. In addition, if the drainage openings are of a small size, it has the further advantage that when the last one of all the drainage openings is blocked by bladder wall tissue when complete voiding (no residual urine in the bladder) is reached, marginal urinal flow through that last to be blocked drainage opening is decreased to a level where abrupt closure of that last opening only causes a minor negative pressure pulse to occur.

During use of an intermittent catheter of the prior art, blocking of the drainage openings by bladder wall tissue may occur, probably due to the inflow sucking the bladder wall tissue towards the drainage opening, as described above. As documented in the tests described below and illustrated in the figures relating to prior art catheters, significant amounts of bladder wall tissue may enter into the inner lumen of the catheter and be trapped in the drainage openings due to the suction of bladder tissue. It is contemplated that this is because, the negative pressure pulse results in the drainage opening being clogged as described above and the once, the drainage opening is clogged, the pressure difference between the bladder wall and the pressure in the inner lumen gradually deforms the bladder wall such that it enters into the drainage opening. If clogging of the drainage openings reduces the urine flow remarkably or entirely, the user may then try to move the catheter up or down or rotate the catheter in order to reposition the drainage openings to regain flow. The user may also withdraw the catheter in the belief that the bladder is empty because the urine flow has stopped. The risk of influencing the bladder wall tissue due to movement of the catheter can be reduced by preventing trapping of bladder wall tissue in the drainage openings.

As described above, the contact between the bladder wall and the drainage openings of a catheter of the prior art can cause the bladder wall tissue to block the drainage openings and thereby reduce or entirely stop the urine flow. Undue blockage of the drainage openings of an intermittent catheter may lead users to withdraw the catheter in the belief that the bladder is empty since the flow has stopped or has diminished remarkably. If catheter users for this reason abort the voiding procedure prematurely, residual urine may remain in the bladder. An intermittent catheter with multiple small drainage openings according to the disclosure prevents premature blockage of the drainage openings, thereby securing urine flow until the bladder is empty. An intermittent catheter as disclosed herein with multiple drainage openings thus secures that catheter users are not falsely led to believe that the bladder is empty and thereby terminate the voiding procedure prematurely, resulting in leaving residual urine in the bladder.

Drainage openings in intermittent catheters, including the ones described herein are sometimes in the art referred to as eyelets or eyes. The drainage openings in the intermittent catheters in this disclosure have a closed loop circumference and may be circular, oval, square, triangular and any other closed loop shape. In embodiments, the drainage openings are star-shaped.

When a catheter is inserted, only a certain length is inserted to ensure drainage of the bladder and avoid discomfort. Ensuring that the urine inflow to the catheter is distributed as much as possible along the surface inserted into the bladder lowers the risk of clogging and by that residual urine together with discomfort due to abruption of flow. Placing multiple smaller openings on the part of the catheter inserted and intended for drainage, ensures a good distribution of the flow. This is opposite the prior art catheters, in which only two large openings are inserted into the bladder, leading to a poorly distributed flow.

In the following, whenever referring to a proximal end of an element of the invention, the referral is to the end adapted for insertion. Whenever referring to the distal end of an element, the referral is to the end opposite the insertion end. In other words, the proximal end is the end closest to the user, when the catheter is to be inserted and the distal end is the opposite end - the end furthest away from the user when the catheter is to be inserted.

The longitudinal direction is the direction from the distal to the proximal end. The transverse direction is the direction perpendicular to the longitudinal direction, which corresponds to the direction across the catheter.

The intermittent urinary catheter according to the disclosure comprises a main tubular portion extending from a tip portion in the proximal insertion end to a distal outlet end to the proximal end. The tubular portion can be cylindrical or conical. In embodiments, the tubular portion has an oval cross-section. The tubular portion is configured for providing urine flow through the intermittent catheter from a drainage portion to the distal end. A closed tip portion with a closed tip is positioned in the proximal end of the catheter and is provided as a rounded closed end of the tube constituting the main tubular portion of the catheter. The drainage portion of the tubular portion will typically be in the proximal portion of the tubular portion. In embodiments, the drainage portion includes multiple drainage openings providing for flow of urine between the outside of the catheter and an inside lumen of the tubular portion. In embodiments, the drainage portion is longer than the typical flow zone on a prior art catheter, where the flow zone is defined as the length from the distal edge of the distal eyelet to the proximal edge of the proximal eyelet. In embodiments, the intermittent catheter comprises a connector in the distal end. In an embodiment the connector comprises a flared end of the catheter so that the diameter of the connector increases with respect to the tubular portion. In embodiments, the intermittent catheter comprises a handle in the distal end, which has a length allowing the user to manipulate the catheter.

Usually, intermittent urinary catheters are from size 8 FR to size 18 FR. FR (or French size or Charriere (Ch)) is a standard gauge for catheters approximately corresponding to the outer circumference in mm. More accurately, the outer diameter of the catheter in mm corresponds to FR divided by 3. Thus 8 FR corresponds to a catheter with an outer diameter of 2.7 mm and 18 FR corresponds to a catheter with an outer diameter of 6 mm.

Intermittent urinary catheters according to the disclosure can be provided with lubrication to ease insertion into the urethra, one example of which includes a hydrophilic coating on the catheter to allow for a low-friction insertion.

The hydrophilic coating may be provided only on an insertable part of the catheter. The hydrophilic surface coating is of the kind which, when hydrated or swelled using a swelling medium, reduces the friction on the surface area of the catheter which is intended to be inserted into the lower urinary tract of a user corresponding to the insertable part of the catheter.

An intermittent hydrophilic coated urinary catheter differs from another type of catheters known as indwelling catheters in that the hydrophilic surface coating of an intermittent hydrophilic coated catheter is not suitable for indwelling use, because the surface coating tends to stick inside the mucosa of the urethra if left inside the body for a period exceeding 5-20 minutes. This is due to the hydrophilic coating transforming from being highly lubricious when fully wetted (95% weight water) to being adhesive when the hydration level of the coating is reduced (<75% weight water).

In the context of this disclosure, "pressure" means pressure difference, not absolute pressure if nothing else is mentioned. This means that "pressure" is indicated as a pressure difference between the point of measurement and the ambient pressure, unless indicated otherwise.

There are several factors determining the flow and pressure in an intermittent urinary catheter during voiding. One factor is the resistance to flow in the tubular portion of the catheter and another factor is the resistance to flow in the drainage openings. These two factors together will provide a combined resistance to flow. In the context of this disclosure, the resistance to flow in the tubular portion is known as R1 and the resistance to flow in the drainage openings is known as R2. The combined effect is known as R.

The flow and pressure in an intermittent catheter depends on the pressure difference between the bladder and the ambience as well as on the flow through the catheter. The bladder pressure depends on the detrusor pressure and the abdominal pressure. The volumetric flow through the catheter will provide a suction effect on the urine in the bladder because the urine, being mostly water, is incompressible and thus the volume flowing out of the catheter will correspond to the volume flowing into the catheter.

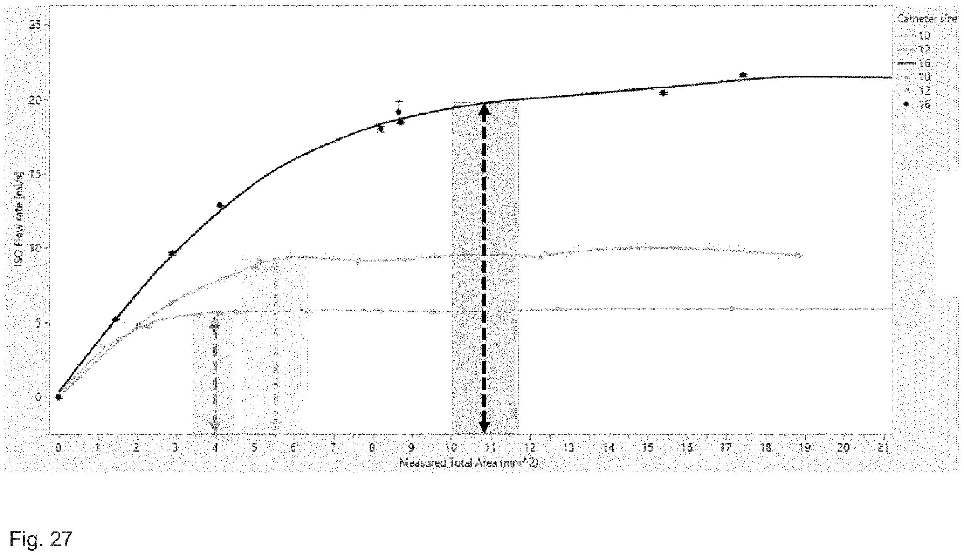

The inflow into the intermittent urinary catheter will increase with the number of drainage openings of a certain size until a maximum flow-rate is reached. In other words, the inflow will increase with the total cross-sectional area of the drainage openings as described below. The maximum flow-rate is determined by the tubular flow resistance in the lumen of the catheter, which at a certain point becomes dominant. This is further illustrated in figure 27, where Q, the volumetric flow rate, is shown as a function of the total cross-sectional area of the drainage openings. The figure illustrates that Q increases up to a certain point, depending on the lumen of the catheter and from there, the total cross-sectional area seems to lose influence on the flow. If drainage openings of a certain size, e.g. 0.7 mm in diameter, is used, then there would be a similar dependency between Q and the number of drainage openings. This means that adding further drainage openings will not lead to an increase in the volumetric flow-rate.

The suction ability is related to the volumetric flow rate through the catheter. The relation between suction ability and the volumetric flow rate is due to the volumetric flow being dependent (amongst others) on the difference in pressure from the inlet of the flow to the outlet of the flow. During draining with an intermittent catheter this pressure difference corresponds to the loss of static pressure between the bladder-pressure and the lumen-pressure at the drainage openings. The resistance to the flow equals the pressure loss in the catheter divided by the volumetric flow rate.

$$R_1 = \frac{\Delta P}{Q} = \frac{8\mu \cdot L}{\pi \cdot a^4}$$

In the above equation, R1 is the resistance to flow, ΔP is the pressure loss, Q is the volumetric flow rate, $\mu$ is the viscosity in the fluid, L is the length of the flow and a is the area over which, the flow occurs - in the present case, the cross-sectional area of the lumen of the catheter. The volumetric flow rate of the lumen can then be expressed as follows:

$$Q = \frac{\Delta P \cdot \pi \cdot a^4}{8\mu \cdot L}$$

The above equation can be used to describe the flow in a lumen of a tubular portion of the catheter.

The inflow from the bladder into the lumen of the catheter is, amongst others, controlled by the size of the drainage openings. In a prior art catheter having only two drainage openings positioned longitudinally apart, the urine in the bladder will predominantly be drained through the drainage opening positioned lowest in the bladder. Around 70% or 80% of the urine will be drained through the drainage opening closest to the bladder neck, when the catheter is inserted into the bladder.

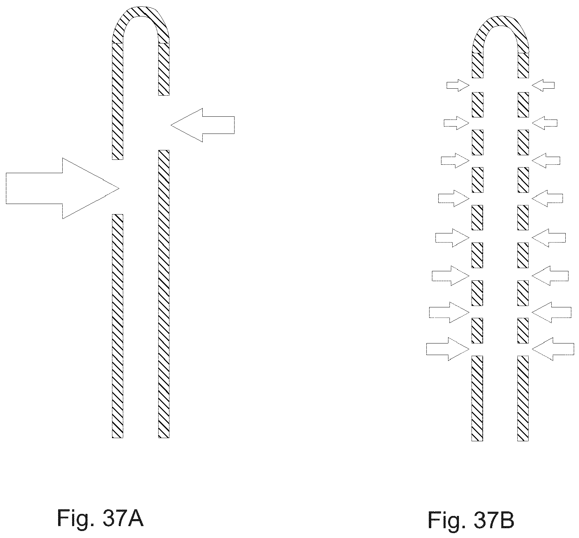

In an intermittent urinary catheter as described herein and having multiple drainage openings, the urine will be more evenly drained, as illustrated in figure 37. The flow rate through the drainage openings positioned closer to the bladder neck (the lower drainage openings) will only be slightly larger than the flow rate through the other drainage openings. This is illustrated in the figure, the arrows indicating the order of the flow into each drainage opening. An intermittent urinary catheter with 16 drainage openings is illustrated in the figure. An intermittent urinary catheter having more drainage openings will have even less difference between the flow rate of the inflow into the upper eyelets and the flow rate of the inflow into the lower eyelets.

One of the determining factors, as mentioned above, is the viscous friction between the urine and the catheter wall through which, the drainage opening extends. In other words, R2. This means that the surface area of each opening influences the inflow from the bladder to the lumen. The surface area is determined as follows:

$$A_{surface} = Circumference \cdot t$$

Where t is the thickness of the catheter wall. A prior art catheter having two larger drainage openings (2.5 by 1.1 mm) will have a total inflow area (see below) of 5.5 mm$^2$. An intermittent urinary catheter having 44 drainage openings each with a diameter of 0.4 mm will also have a total inflow area of 5.5 mm$^2$. This means that these two catheters should be able to drain the same amount of urine over time. However, the total surface area of the drainage openings is markedly different. For the prior art catheter, the total surface area is 14 mm (the sum of the circumference of two drainage openings of 2.5 by 1.1 mm) times the catheter thickness, whereas the same value for the intermittent urinary catheter having 44 small drainage openings is approximately 55 mm (the total circumference of 44 drainage openings each having a circumference of 1.25 mm) times the catheter thickness. If the catheter thickness is the same, this illustrates that the total surface area of the intermittent urinary catheter having 44 small drainage openings are 3 times the total surface area of a prior art catheter. This means that the resistance due to viscous friction is markedly higher in the intermittent urinary catheter with small drainage openings - and thereby, the more proximal drainage openings are "forced" to contribute to a larger extent to give a more even distribution of flow and pressure across the external surface of the catheter as well as in a distance close to the catheter. In other words, the flow resistance caused by viscous friction between urine and the catheter wall, through which the drainage openings extend, is increased as the size of the drainage openings decreases. This is due to the fact that there are more drainage openings - hence a larger area, which the urine is in contact with during voiding. This is in spite of the total drainage area. Changing the distance, size and shape of openings with respect to position higher or lower on the catheter enables to design how evenly the flow is distributed. Longer less concentration of openings closer to the bladder neck, will increase the flow higher up on the catheter. In similar ways smaller drainage openings lower down will increase the flow higher up during voiding.

As explained above, an intermittent urinary catheter as in this disclosure and provided with multiple small drainage openings, will distribute the flow and pressure more evenly across the external surface of the drainage portion. During voiding, the drainage openings drain urine, which eventually increases the pressure close to the drainage opening. Thus, bladder tissue getting in close proximity to the drainage openings may be sucked into the drainage opening, as mentioned above.

The magnitude of the negative pressure is related to the flowrate, the size of the drainage opening and the distance to the drainage opening. The distance to the drainage opening is transverse to the longitudinal direction of the catheter. The maximum suction pressure will typically occur in an axial direction with respect to the drainage opening. In this context, the maximum suction pressure is defined as the nominal value of the determined pressure. This means that maximum suction pressure is the nominal value of the lowest value of the pressure determination. On a pressure curve, this corresponds to the largest peak. There will be areas on the surface within the drainage portion, where the flow and suction pressure will be minimal. Consistent with the definition of maximum suction pressure, the minimal suction pressure is the nominal value of the determined pressure, meaning that the minimal suction pressure is the nominal value of the highest value of the pressure determination. On a pressure curve, this corresponds to the point closest to zero. This will typically be at a certain distance from the drainage openings, and if the catheter is provided with multiple drainage openings, it will typically be between two drainage openings in either direction. The difference between the maximum suction pressure and the minimal suction pressure will in this disclosure be known as the pressure ratio.

Embodiments relate to an intermittent urinary catheter having a tip portion with a tip in a proximal insertion end of the catheter, a tubular portion extending from the tip portion to a distal outlet end, the tubular portion having an inside lumen and drainage portion provided with drainage openings configured for allowing urine to enter the inside lumen, the drainage openings being configured to reduce the difference in a pressure ratio between maximum suction pressure and minimal suction pressure, where the maximum and minimal suction pressure are determined in a distance of 1 mm from the drainage portion of the catheter. The distance is in these embodiments set to be 1 mm externally of the catheter in either direction. For a normal cylindrical catheter this means that the suction pressure is determined on a virtual cylinder corresponding in length to the drainage portion and having a cross-section, which is 1 mm larger in all directions from the catheter.

This pressure ratio is considered to be a good measure for how well the flow is distributed, and by that how well the pressure zone avoids suction of the bladder wall. Thus, an intermittent catheter as above provide an optimized flow and pressure distribution on an external surface of the drainage portion of a catheter. It is found that during voiding, no matter the speed of the flow in total or pressure conditions in general, the ratio should be less than 50 and preferable less than 20 or even less than 10.

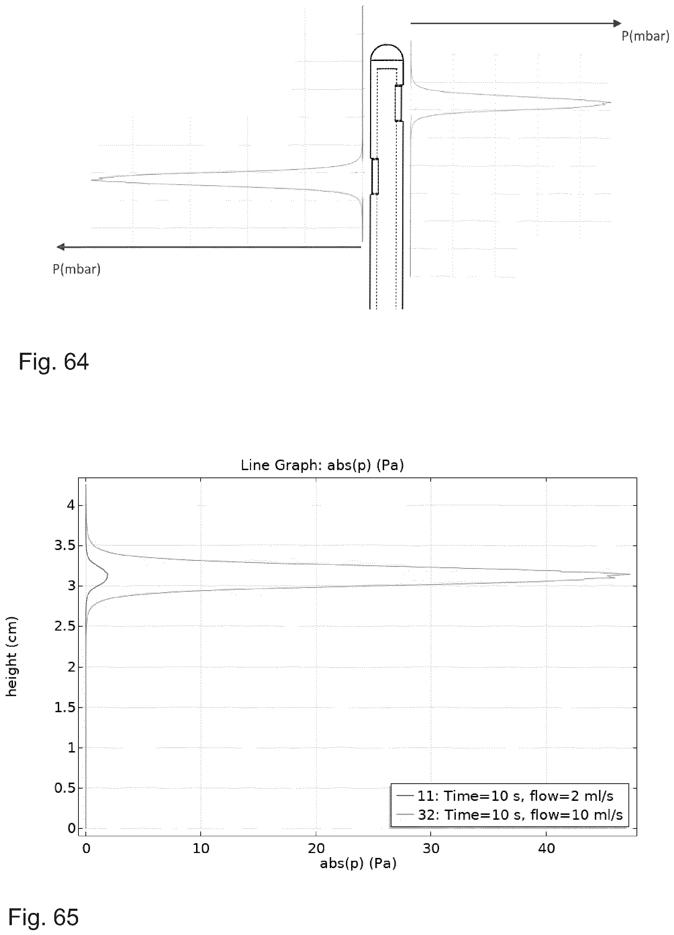

Catheter flow simulations and other tests were conducted, as described below, and show that having fewer larger drainage openings (such as two standard eyelets) produces a locally high suction pressure when measured 1 mm from the drainage opening - see Figure 64.

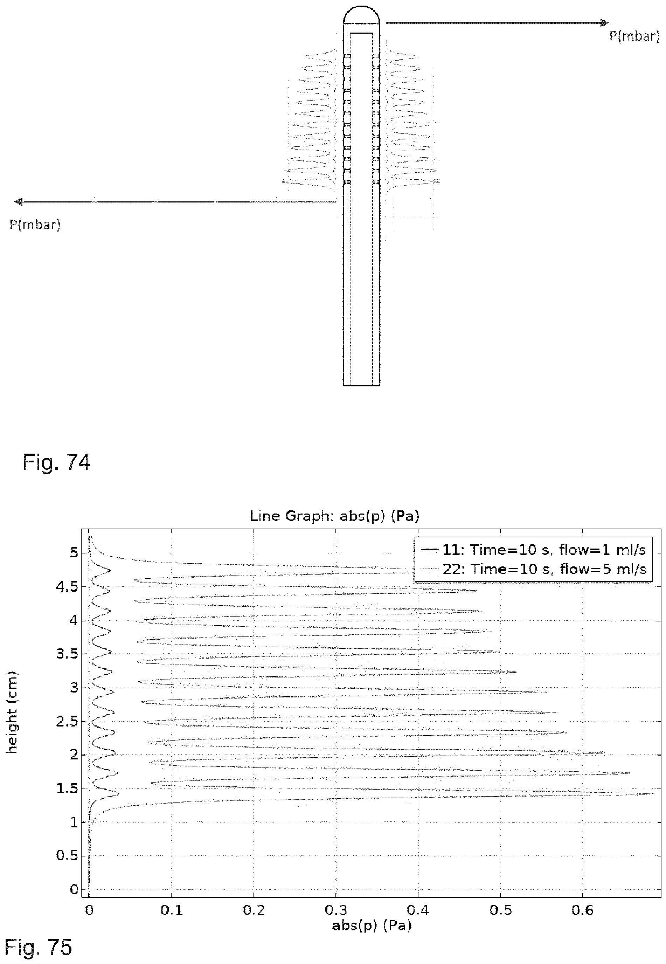

Simulations also show that if the catheter has few larger drainage openings then there will be surface areas within the flow zone or drainage portion, where the minimal suction pressure reaches zero or very close to zero. This means that a catheter having few larger drainage openings will provide a very large pressure drop. Using multiple small drainage openings show that the flow and pressure is much more evenly distributed along longitudinal rows of drainage openings as well as for the entire surface around the catheter in a certain distance, see Figure 74. Consequently, the suction pressure in the urine inflow is much more evenly distributed and reduces the risk of dragging bladder tissue into the drainage openings.

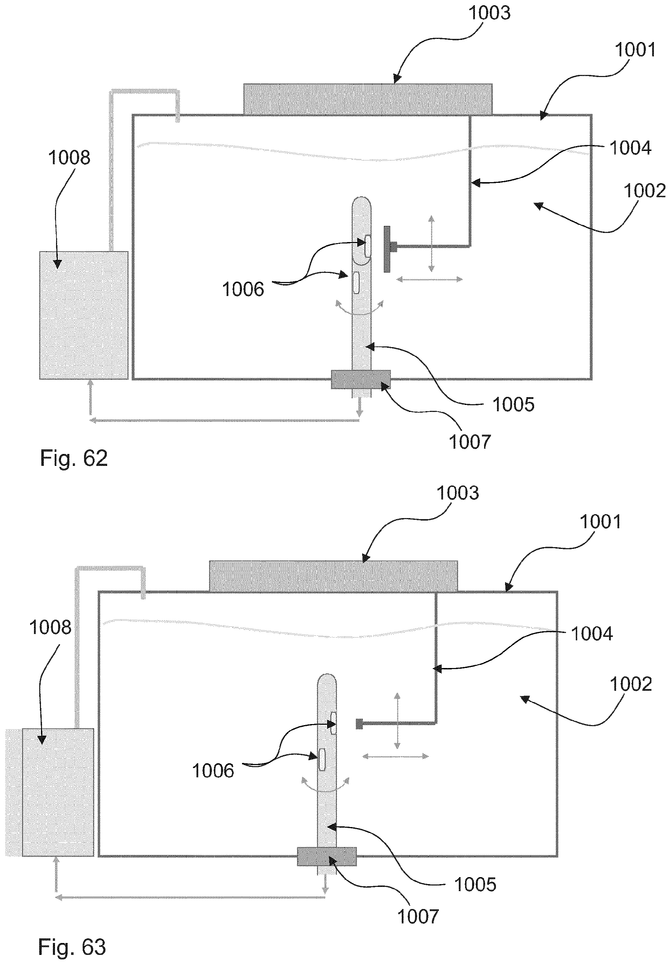

For practical reasons, the drainage openings may be positioned in longitudinal extending rows along the catheter axis. Thus, the distribution of the flow is mainly along these rows, and the internal distance between the different openings may be adjusted to have a better distribution of the flow in comparison to the existing catheter having e.g. one larger eyelet on each side. In order to minimize the risk of dragging bladder tissue into the eyelets, the distance, number and form of the drainage openings should be so that when determining the resulting flow or suction pressure profile along a single row of openings, the difference in the resulting pressure ratio should not exceed a factor of 70. This is determined 1 mm radially away from the surface of the catheter - along the row of drainage openings. In other words, the determination is done along a virtual row positioned 1 mm externally of a row of drainage openings. The determination may be done as in Example 7 and shown in Figure 63.

For practical reasons it is also found that at a factor of less than 50, less than 20 or 10 or even 5 in pressure difference, it is still feasible to manufacture a catheter which is less prone to drag bladder tissue into the drainage openings.

In some embodiments the drainage openings are not placed in rows, which increases the freedom to form and position the drainage openings for minimizing the risk of dragging the bladder tissue into the eyelets. Here, the pressure ratio should still be less than a factor 70 when measured 1 mm from the surface of the catheter in the drainage portion - but both along the catheter axis in any position on the circumference. Again, it is also found that if the factor in pressure is less than 50, 20, 10 or even 5, the catheter is less prone to drag tissue into the drainage openings.

Thus embodiments relate to an intermittent urinary catheter having a tip portion with a tip in a proximal insertion end of the catheter, a tubular portion extending from the tip portion to a distal outlet end, the tubular portion having an inside lumen and drainage portion provided with drainage openings configured for allowing urine to enter the inside lumen, a number and size the of drainage openings being configured for providing an even distribution of inflow through the drainage openings.

The even distribution of inflow is defined as being within a factor 70 between a maximum inflow transversely with respect to a drainage opening and a minimal inflow at any point between the drainage openings. The distribution of inflow can be determined in a distance of 1 mm from the catheter in either direction.

Further embodiments relate to the distribution being within a factor 50 between a maximum inflow transversely with respect to a drainage opening and a minimal inflow at any point between the drainage openings. Further embodiments relate to the factor being 20, 10 or as low as 5.

Embodiments relate to an intermittent urinary catheter having a tip portion with a tip in a proximal insertion end of the catheter, a tubular portion extending from the tip portion to a distal outlet end, the tubular portion having an inside lumen and drainage portion provided with drainage openings configured for allowing urine to enter the inside lumen, a number and size of the of drainage openings being configured for providing a non-interrupted flow.

By non-interrupted flow is meant a flow that is continuous until the bladder is empty, i.e. a substantially continuous flow. This means that the flow may slow down - but will resume by itself without re-positioning of the catheter. Providing a non-interrupted flow ensures that the user leaves the catheter in the bladder until the bladder is empty. Thereby the risk of leaving residual urine in the bladder following a catheterisation is alleviated - or at least reduced to a great extent.

Embodiments relate to an intermittent urinary catheter having a tip portion with a tip in a proximal insertion end of the catheter, a tubular portion extending from the tip portion to a distal outlet end, the tubular portion having an inside lumen and drainage portion provided with drainage openings configured for allowing urine to enter the inside lumen, a number and size of the drainage openings being configured for preventing clogging of the drainage openings.

The inflow of urine through the multiple drainage openings depends on the total sum of the cross-sectional area of all of the drainage openings (the total inflow area) and the pressure gradient between the drainage openings and the outlet from the catheter at the distal end, as explained above. The total sum of cross-sectional area of the multiple drainage openings (the total inflow area) has to be large enough to provide for an adequate inflow of urine, otherwise emptying of the bladder would take too long and thus be an inconvenience to the user of the intermittent catheter. Each drainage opening provides a certain resistance to inflow of urine, which resistance depends inter alia on the cross-sectional area of the drainage opening and the thickness of the catheter material at the drainage opening.

Embodiments relate to the total sum of the cross-sectional area of the multiple drainage openings being larger than the cross-sectional area of the inside lumen of the catheter just distally of the drainage openings. By just distally of the drainage openings is meant within 5 mm in longitudinal distal direction from the most distal drainage opening.

Embodiments relate to the tubular portion defining a convex outer surface, and wherein the total inflow area of the drainage openings in the convex outside surface of the tubular portion is larger than a cross-sectional area of the inside lumen of the catheter in a cross section perpendicular to a longitudinal direction of the tubular portion at a position distally of the drainage openings.

In an embodiment, the total sum of the cross-sectional area of the multiple drainage openings (the total inflow area) is larger than twice the cross-sectional area of the inside lumen of the catheter just distally of the drainage openings. The total inflow area of the drainage openings is provided in a convex outside surface of the tubular portion. Providing such a large total inflow area ensures that the resistance of flow at the drainage openings will not hinder a filling of the inside lumen of the catheter. Therefore, the inflow through the drainage openings into the inner lumen does not limit the flow through the intermittent catheter.

Further embodiments relate to the total sum of the cross-sectional area of the multiple drainage openings (the total inflow area) being at least three times larger than the cross-sectional area of the inside lumen of the catheter.

Embodiments relating to the total inflow area in a convex outside surface of the tubular portion being at least equal to or higher than the cross-sectional area of an inside lumen of the tubular portion may relate to a catheter having a cylindrical tubular portion. In this case, the cross-sectional area of the inside lumen is constant through-out the length of the catheter. However, these embodiments may also relate to a catheter having a conical tubular portion. In this case, the cross-sectional area increases along the length. In this case, the total inflow area should be compared to the cross-sectional area of the inside lumen just distally of the most distal drainage openings, i.e. within 5 mm in the distal direction of the most distal drainage opening.

Embodiments relate to the number of drainage openings being higher than required for filling the lumen just distally of the drainage openings. This is to be understood such that, depending on the size of the individual drainage openings, a certain number of drainage openings is required to provide a total inflow area corresponding to the cross-sectional area of the lumen distally of the drainage openings. This number of drainage openings is in this disclosure referred to as first predetermined number of drainage openings. Thus, embodiments relate to the number of drainage openings being higher than a first predetermined number of drainage openings.

Embodiments relate to an intermittent urinary catheter as defined above and provided with multiple drainage openings configured for providing a total inflow area exceeding an inside lumen in the catheter just distally of the most distal of the drainage openings.

Embodiments relate to an intermittent urinary catheter having a tip portion with a tip in a proximal insertion end of the catheter, a tubular portion extending from the tip portion to a distal outlet end; the tubular portion having an inside lumen and a drainage portion provided with drainage openings configured for allowing urine to enter the inside lumen, wherein the largest dimension of any of the drainage openings is less than 1mm and the total inflow area of the drainage openings is larger than the cross-sectional area of the inside lumen of the catheter.

When the total inflow area exceeds the inside lumen of the catheter or the number of drainage openings is higher than what is required for filling the inside lumen, then it is ensured that at least one drainage opening is always available for providing inflow. This is because the inflow is less than what the drainage openings are able to drain - and therefore at least one drainage opening will be able to provide further inflow, should another one of the drainage openings being contemporarily blocked by bladder tissue. This means that the flow through the catheter will be continuous until the bladder is empty. Thereby the risk of leaving residual urine in the bladder is substantially alleviated.

Embodiments relate to an intermittent urinary catheter having a degree of perforation of between 0.02-0.3 Embodiments relate to an intermittent urinary catheter having a degree of perforation of 0.4-0.6. The degree of perforation is defined as the total sum of the cross-sectional area of the multiple drainage openings divided by the surface area of the catheter at the portion of the catheter including the drainage openings.

$$\eta = \frac{\sum A_{openings}}{A_{catheter}} = \frac{\sum\left(\pi \cdot r_{openings}^2\right)}{\pi \cdot l \cdot d} = \frac{\sum r_{openings}^2}{l \cdot d}$$

In the above equation, $\eta$ is the degree of perforation, $A_{openings}$ is the cross-sectional area of a drainage opening, $A_{catheter}$ is the surface area of the catheter portion where the drainage openings are. Furthermore, $r_{openings}$ is the radius of an opening, $l$ is the length of the drainage zone and $d$ is the diameter of the catheter.

A degree of perforation of 0.02-0.3 is advantageous if less than 50 drainage openings are used, i.e. such as 12, 24 or 48. A degree of perforation of 0.4-0.6 is advantageous if 100 or drainage openings are used, such that as 100, 144, 160 or 200 drainage openings.

Embodiments relate to an intermittent urinary catheter having a first degree of perforation of 0.4-0.6 in a proximal portion of the drainage portion and a second degree of perforation of 0.1-0.3 distally thereof. Embodiments relate to an intermittent urinary catheter having a first degree of perforation of 0.5-0.7, a second degree of perforation of 0.3-0.5 and a third degree of perforation of 0.1-0.3.

Different degrees of perforation is an advantage because it provides a catheter where the drainage portion is divided into two or more zones with different degrees of perforation. Thereby the inflow can be higher in one zone than in another, depending on the positioning of the catheter in the bladder.

Other embodiments relate to an intermittent urinary catheter having a first degree of perforation of approximately 0.02 in a first proximal portion of the drainage portion and a second degree of perforation of approximately 0.01 in a second portion of the drainage portion, the second portion being located distally of the first proximal portion.

Embodiments relate to a catheter comprising an intermittent urinary catheter having a tubular portion extending from a proximal insertion end to a distal outlet end, with the tubular portion formed to include a lumen adapted to transport urine through the intermittent urinary catheter; a drainage area provided on an exterior surface of the tubular portion, where the drainage area includes a plurality of drainage openings that combine to provide an open drain area; wherein the drainage are is defined by a length measured form a proximal edge of a proximal most one of the plurality of the drainage openings that is closest to the proximal insertion end to a distal edge of a distal most one of the plurality of the drainage openings that is closest to the distal outlet end multiplied by a circumference of the tubular portion measured within the length of the drainage area; wherein a ratio of the open drain area to the drainage area on the exterior surface of the tubular portion is in a range from 0.05 to 0.7.

Consistent with the above description, embodiments provide a ratio of the open drain area to the total possible drainage area on the exterior surface of the tubular portion in a range from 5% to 70%. For the embodiments with the ratio of the open drain area to the total possible drainage area on the exterior surface of the tubular portion in the range from 51% to 70%, this means that there is more open area on the tubular portion than closed area.

Embodiments relate to the ratio of the open drain area to the drainage area on the exterior surface of the tubular portion being in the range from 0.05 to 0.20
Embodiments relate to the ratio of the open drain area to the drainage area on the exterior surface of the tubular portion being in the range from 0.2 to 0.5.

Embodiments relate to the ratio of the open drain area to the drainage area on the exterior surface of the tubular portion being in the range from 0.4 to 0.7.

Embodiments relate to a catheter comprising an intermittent urinary catheter having a tubular portion extending from a proximal insertion end to a distal outlet end, with the tubular portion formed to include a lumen adapted to transport urine through the intermittent urinary catheter; a drainage area provided on an exterior surface of the tubular portion, where the drainage area includes a closed surface area and an open surface area, where the open surface area of the drainage area allows urine to enter the lumen; wherein the drainage area is defined by a length measured as a longitudinal distance from a proximal most edge of the open surface area to a distal most edge of the open surface area multiplied by a circumference of the tubular portion measured within the length of the drainage area; wherein the open surface area comprises from 5% to 70% of the drainage area on the exterior surface of the tubular portion.

Embodiments relate to the open surface area comprising from 20% to 50% of the drainage area on the exterior surface of the tubular portion.

Embodiments relate to the open surface area comprising from 40% to 60% of the drainage area on the exterior surface of the tubular portion

In an embodiment, the largest dimension of an individual drainage opening in an outer convex surface of the tubular portion is less than 1 mm. By largest dimension is meant a diameter in case of a circular drainage opening, the major axis in case of ellipse, the diagonal in case of a rectangular or square opening and so forth. In other words, the largest dimension means the largest of the dimensions across the opening between two oppositely located points on the perimeter of the opening at an outer convex surface of the tubular portion. In a related embodiment, each of the drainage openings has a cross-sectional area of less than 0.8 mm$^2$.

Thereby, it is secured that a negative pressure no larger than 50 mBar when measured under 10 cm $H_2O$ can occur, thus the influence to the bladder wall tissue is significantly reduced as compared to prior art catheters having a few (such as two) large drainage openings.

In an embodiment, the largest dimension of any one individual drainage opening in an outer convex surface of the tubular portion is less than 0.7 mm. In a related embodiment, each of the individual drainage openings has a cross-sectional area of less than 0.4 mm$^2$. Thereby, it is secured that the negative pressure can be no more than 40 mBar when measured under 10 cm $H_2O$.

In an embodiment, the largest dimension of any one individual drainage opening in an outer convex surface of the tubular portion is less than 0.5 mm. In a related embodiment, each of the individual drainage openings has a cross-sectional area of less than 0.2 mm$^2$.

In an embodiment, the number of drainage openings are more than 20.

Thereby, the likelihood of all drainage openings being blocked at once is significantly reduced.

In embodiments, the number of drainage openings can be significantly higher, for example more than 200 or even around 260 drainage openings. The number can also be around 100, 120 or 150 - or close to 200 such as 180.

Embodiments relate to an intermittent urinary catheter wherein the catheter is a CH10, each drainage opening has largest dimension in an outer convex surface of the tubular portion of approximately 0.4 mm, and the number of drainage openings is larger than 32. Such a catheter provides for adequate inflow into the lumen of the catheter such that each drainage opening contributes to the draining, but at least one drainage opening is always left open. By approximately 0.4 mm is meant between 0.35 and 0.45 mm.

Other embodiments relate to an intermittent urinary catheter, wherein the catheter is a CH12, each drainage opening has largest dimension in an outer convex surface of the tubular portion of approximately 0.7 mm, and the number of drainage openings is larger than 15. By approximately 0.7 mm is meant between 0.65 mm and 0.75 mm.

Embodiments relate to an intermittent urinary catheter as in any of the preceding claims, wherein each one of the drainage openings extends transversely to a longitudinal direction of the catheter. By extending transversely is meant that a central axis of the drainage opening is substantially perpendicular to the longitudinal axis of the catheter meaning within 20 degrees in either direction.

In an embodiment, the drainage portion has a length of 4 cm in a longitudinal direction of the intermittent catheter. This provides for good emptying of the bladder. The drainage part is positioned distally of the closed tip portion, thus if the closed tip portion is less than 2 cm in a longitudinal direction, the drainage portion is within the most-proximal 6 cm of the catheter. This is a common insertion length of intermittent catheters into a bladder - thus having the drainage portion positioned inside the bladder provides for a large cross-sectional area of the multiple drainage openings being inside the bladder and hence a good and fast draining of the bladder. A drainage portion of approximately 4 cm may be useful for both male and female catheters. By approximately 4 cm is meant between 35 mm and 45 mm, such as 40 mm, 37 mm or 42 mm.

In an embodiment, the drainage portion has a length of 10 cm in a longitudinal direction of the intermittent catheter. This provides an enhanced security for emptying of the bladder as there will be drainage openings positioned in the lower part of the bladder, at the bladder neck. Typically, the intermittent catheter will be inserted 5-6 cm into the bladder, thus in these embodiments the drainage part will extend into a portion of the urethra as well as being in the bladder. A catheter with a drainage portion of 10 cm or more is particularly useful for male catheters. Other embodiments relate to a drainage portion having a length of approximately 8 cm, meaning between 75 mm and 85 mm, such as 77 mm, 80 mm or 82 mm.

In an embodiment, the drainage portion has a length of 15 cm in a longitudinal direction of the intermittent catheter. This provides an enhanced security for emptying of the bladder. This is particularly beneficiary for users who have a tendency of inserting their intermittent catheter too far into the bladder, probably because they have no sense of feeling during insertion of the catheter.

Embodiments relate to a drainage portion having a length of approximately 2 cm, meaning between 15 and 25 mm. Such a short drainage portion is particularly useful for female catheters, where the urethra is quite short. A short drainage portion reduces the risk of urine flowing out through the drainage openings, in case some of the drainage openings are situated outside the urethra.

In embodiments, the drainage portion is divided into a first drainage portion and a second drainage portion. Embodiments relate to the second drainage portion being positioned distally of the first drainage portion. Embodiments relate to the first drainage portion being configured for being positioned in the bladder during use. Embodiments relate to the second drainage portion being configured for being positioned towards the bottom of the bladder and in the upper part of the urethra during use.

In an embodiment, the multiple drainage openings are positioned in a scattered manner in the longitudinal direction as well as around the circumference of the intermittent catheter.

In an embodiment, the multiple drainage openings are positioned in four longitudinal rows with 90 degrees between them around the circumference of the intermittent catheter.

In an embodiment, the multiple drainage openings are positioned in 6 (six) longitudinal rows with 60 degrees between each row around the circumference of the intermittent catheter.

In an embodiment, the multiple drainage openings are positioned in 8 (eight) longitudinal rows with 45 degrees between each row around the circumference of the intermittent catheter.

In an embodiment, the multiple drainage openings are positioned in two longitudinal rows with 180 degrees between the rows around the circumference of the intermittent catheter.

In an embodiment, the multiple drainage openings are positioned in two pairs of parallel rows with 180 degrees between pairs of rows around the circumference of the intermittent catheter.

In an embodiment, the multiple drainage openings are dispersed according to a helical distribution around the circumference of the intermittent catheter.

Having an increased number of directions provide for better inflow and decreased risk of bladder tissue blocking contact with all drainage openings. As shown in Example 6 and figures 64 to 77, more than two rows provide a better distribution of flow and pressure around the circumference of the intermittent urinary catheter.

In an embodiment, the closed tip portion of the intermittent catheter is a Nelaton tip, where the proximal end is simply closed off and provides a half-spherical closed end.

In embodiments, the closed tip portion is integrally moulded with the tubular portion- either as a one-component or as a two-component moulding. Alternatively, it can initially be provided as a separate element and then attached to the tubular portion, e.g. by welding or adhering.

In an embodiment, the closed tip portion is a flex tip. In this type of embodiment, the closed tip portion of the intermittent urinary catheter comprises, from the proximal end of the closed tip portion, a proximal portion having a bulb-shaped portion with a diameter close to or exceeding the diameter of the tubular portion of the catheter, an intermediate portion, where the outer catheter diameter is decreased with respect to the diameter of the remaining part of the catheter, and a distal portion transitioning into the tubular portion of the intermittent urinary catheter.. Alternatively, the bulb-shaped portion can have a diameter that is slightly less than the diameter of the tubular portion of the catheter. In embodiments, the bulb-shaped portion is close to spherical in shape or alternatively slightly elongated and shaped as an olive or droplet. This type of closed tip portion can be useful for male users to guide the catheter around the bend in the urethra at the prostate.

In embodiments, the intermittent urinary catheter is made of a polyurethane material (PU) or polyvinyl chloride (PVC) or poly-olefins such as a polyethylene (PE). Other suitable materials include silicone materials, latex material, styrenic block copolymers, TPS (TPE-s) (thermoplastic elastomeric materials), thermoplastic vulcanizates, TPV, Thermoplastic copolyester, TPC (TPE-E), thermoplastic polyamides, TPA, (TPE-A).

Embodiments relate to a method of reducing a pressure pulse at the drainage openings in the bladder as a result of blocking of the drainage openings, by using an intermittent urinary catheter as described above.

By a reduction in the pressure pulse is meant that the suction force at the drainage opening is reduced and thereby the suction pressure that may occur due to the drainage openings being blocked is reduced.

Embodiments relate to a method of reducing the pressure pulse to below predetermined threshold value, when tested as described herein.

Related embodiments relate to a method wherein the predetermined threshold value is 350 mBar. Further related embodiments relate to a method wherein the predetermined threshold value is 300 mBar or 200 mBar. A threshold value may be determined as in Example 2 below.

Embodiments relate to a method of providing an intermittent urinary catheter that is configured for emptying without repositioning of the catheter during the draining process.

Another way of saying this that embodiments relate to a method of emptying a bladder by using an intermittent urinary catheter as mentioned above, wherein emptying of the bladder is performed while the catheter is held stationary during the emptying procedure.

Users may find it difficult to try to ascertain how many times they should reposition the catheter to ensure that the bladder is satisfactorily emptied. This problem is alleviated by the method mentioned above.

Related embodiments relate to a use of an intermittent urinary catheter as mentioned above, wherein the use does not require the step of repositioning of the catheter during the emptying procedure. Further related embodiments relate to the use of an intermittent urinary catheter as mentioned above, wherein the catheter is held stationary during the emptying procedure.

Embodiments relate to a method of reducing a drag-force provided by the drainage openings in a distance of 1 mm from the drainage openings to a level below 1 mN during flow in the catheter lumen below 10 ml/s.

Further embodiments relate to reducing the drag-force to a level below 0.6 mN during flow in the catheter lumen below 10 ml/s.

Embodiments relate to a use of an intermittent urinary catheter according to any of the preceding claims, wherein a flow-rate through the catheter exceeds zero ("0") through-out the catheterisation procedure.

Intermittent urinary catheters according to this disclosure provides a continuous flow-rate through-out the voiding of the bladder. Therefore, the user is less prone to inadvertently remove the catheter prior to the bladder being empty.

Embodiments relate to a use of an intermittent urinary catheter as mentioned above, wherein the catheter is inserted into the urethra until the drainage portion reaches the bladder, the catheter is held in position during the draining of urine, and the catheter is removed, wherein the catheter is configured for draining the bladder without repositioning of the catheter during the draining of urine.

Related embodiments relate to the use of an intermittent urinary catheter as mentioned above, wherein the catheter is inserted into the urethra until the drainage portion reaches the bladder, the catheter is held in position during the draining of urine, and the catheter is removed, wherein the catheter is configured for draining the bladder while the catheter is held stationary during the draining of urine.

Embodiments relate to a use of an intermittent urinary catheter, wherein the catheter is configured for reducing the drag-force provided by the drainage openings to a level below 1mN in a distance of 1 mm from the drainage openings, when the flow rate is less than 10 ml/s

**Examples**

**Example 1:**

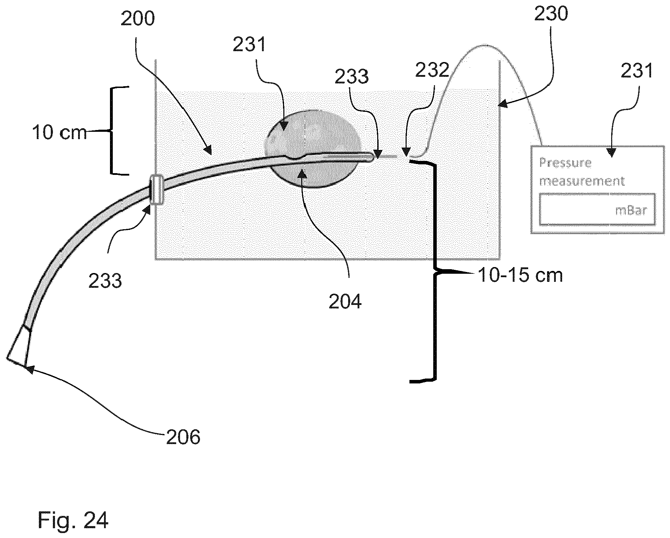

The first tests were performed to compare the level of a pressure pulse between prior art catheters and catheters having small drainage openings with a largest dimension below 1.2 mm. The aim of these first tests is to simulate the situation, where one drainage opening becomes blocked by bladder tissue and the second (last) drainage opening suddenly becomes blocked. Catheters having one small drainage opening (largest dimension below 1.2 mm) were used and compared to a standard prior art catheter provided with two standard sized drainage openings. In the latter case, one drainage opening was prior to testing blocked by a piece of tape. In all tests, the catheter was submerged in a water tank and draining initiated. The test set-up is shown in figures 24 to 26 and mentioned below. The pressure pulse in the inner lumen of the catheter was determined at the moment of blocking the second drainage opening. This corresponds to the situation during a catheterisation, where a first one of the two drainage openings in a prior art catheter is blocked either by bladder tissue or by urethral tissue and the suction through the catheter (caused by the flowing liquid) suddenly provides a blockage also of the second one of the two drainage openings by tissue.

The equipment used for the test is listed here:

- Water tank with hole and O-ring

- 25 L of water
- Catheter with one open drainage opening. If the catheter is provided with two drainage openings, one drainage opening was blocked during the test.
- Waterproof pressure sensor attached to needle
- 5 x 5 cm piece of porcine bladder
- Latex gloves

The testing was done according to the following test-protocol:

- provide a water tank including a sealing adapted for providing a liquid tight sealing around the circumference of a catheter
- Insert the catheter tip into the tank through the liquid-tight sealing, until the one open drainage opening is well inside the water tank
- Let the water begin to flow out through the catheter
- Make sure that there are no air bobbles in the catheter by tapping it
- Insert the sensor-needle into the catheter lumen app. 1 cm from the one open drainage opening
- Make sure that there are no air bubbles in the catheter or in the needle. This is important as even small water bubbles can obscure the pressure readings
- Once there are no air bubbles in the catheter or in the needle, adjust the catheter's position in the water to an immersion depth of 10 cm, meaning that the one open drainage opening is approximately 10 cm below the surface of the water
- Position the portion of the catheter external to the water tank so that the height difference between the one open drainage opening and the catheter connector is around 15-20 cm.
- Put on latex gloves, and take the porcine bladder tissue
- Submerge the tissue in the water
- Start the pressure recording, and make sure to tare the sensor, i.e. set it to zero
- Slowly guide the porcine bladder tissue towards the one open drainage opening
- When the porcine bladder tissue encounters the one open drainage opening, a large (negative) pressure fluctuation occurs in the inner lumen of the catheter
- Note the magnitude of this pressure fluctuation

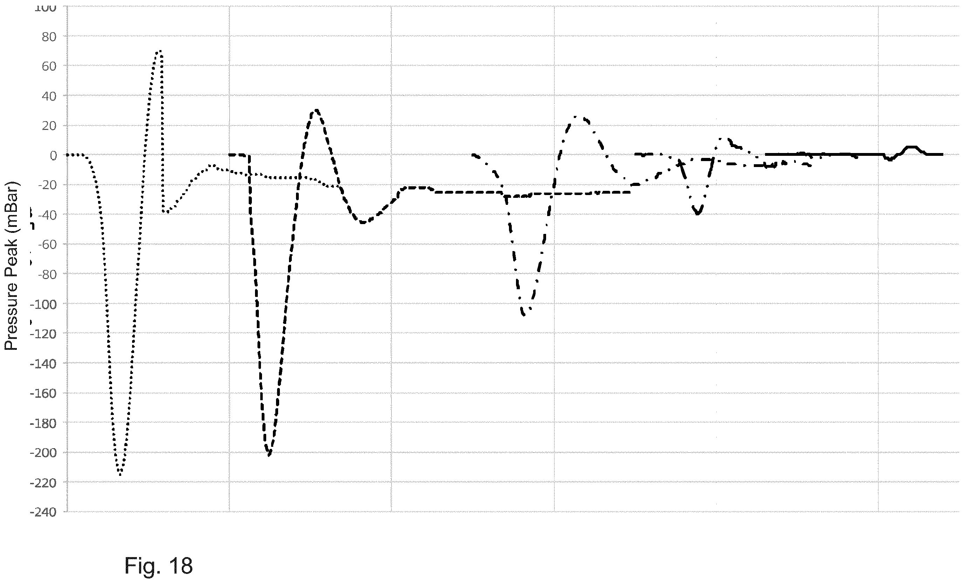

The pressure fluctuation noted corresponds to the pressure pulse in the lumen of the catheter. It will be noted as a (negative) peak on the pressure curve - see examples in figure 18-20.

Some test results are shown in Table 1 below:

**Table 1**

| ID | Largest dimension (mm) | Suction pressure (mBar) |
|---|---|---|
| 1.1 | 0.20 | -1 |
| 1.2 | 0.46 | -8 |
| 1.3 | 0.55 | -15 |
| 1.4 | 0.65 | -15 |
| 1.5 | 0.97 | -44 |
| 1.6 | 3.90 | -200 |

As can be seen from the table above, when the largest dimension of a drainage opening is below 1 mm (ID 1.1-1.5), the suction pressure is significantly reduced compared to a prior art catheter with a drainage opening having a largest dimension of 3.9 mm (ID 1.6). Embodiments of catheters according to the disclosure as in ID 1.1 to 1.5 of Table 1, all have a suction pressure below 50 mBar (below 44 mBar), whereas the prior art catheter in ID 1.6 with a drainage opening having a largest dimension of 3.9 mm has a suction pressure of 200 mBar. Thus a threshold value of suction pressure in a lumen of an intermittent catheter according to this disclosure may be set to 50 mBar, when tested under 10 cm $H_2O$ as described above in Example 1.

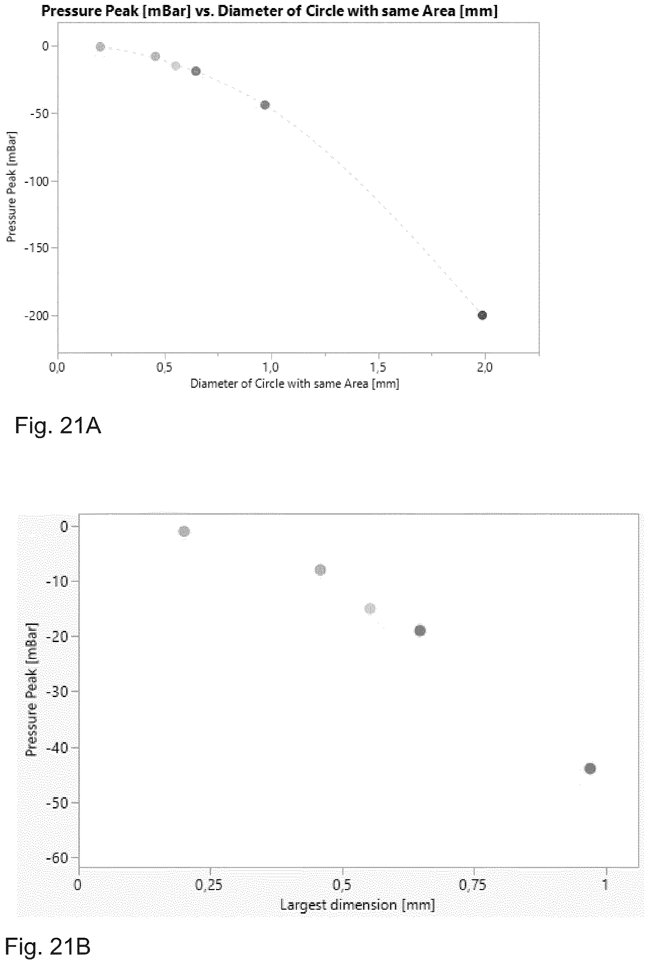

The test results are also illustrated in figures 18, 21A, 21B.

The pressure inside a normal functioning bladder may reach around 400-500mBar (40-50 cm $H_2O$) prior to emptying.

*Example 2:*

The second and third tests was done in a similar fashion - the only difference being that the catheter was submerged under 50 cm of $H_2O$ as opposed to 10 cm. Furthermore, both male and female catheters were tested. The male catheters were tested with a height difference of 25 cm between the drainage opening and the outlet (the connector) and the female catheters were tested with a height difference of 6 cm between the drainage opening and the outlet (the connector).

Results are shown in Table 2 and Table 3 below:

**Table 2**

| ID | Largest dimension (mm) | Suction pressure (mBar) |
|------|------------------------|-------------------------|
| 1.7  | 0.19 | -15 |
| 1.8  | 0.32 | -55 |
| 1.9  | 0.40 | -86 |
| 1.10 | 0.51 | -125 |
| 1.11 | 0.60 | -166 |
| 1.12 | 0.87 | -300 |
| 1.13 | 0.99 | -354 |
| 1.14 | 4.00 | -652 |

**Table 3**

| ID | Largest dimension (mm) | Suction pressure (mBar) |
|------|------------------------|-------------------------|
| 1.15 | 0.19 | -12 |
| 1.16 | 0.32 | -48 |
| 1.17 | 0.40 | -72 |
| 1.18 | 0.51 | -100 |
| 1.19 | 0.60 | -128 |
| 1.20 | 0.87 | -233 |
| 1.21 | 0.99 | -304 |
| 1.22 | 4.00 | -639 |

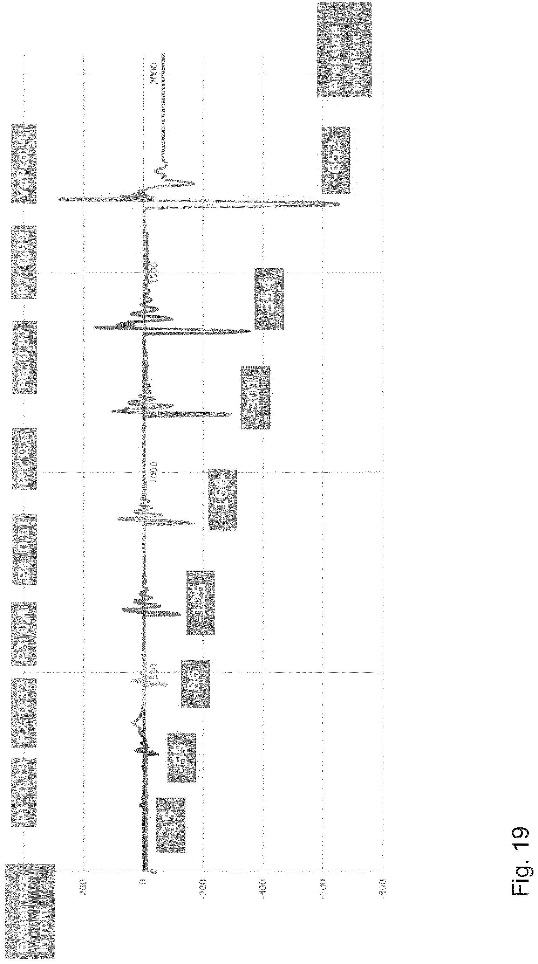

Results are also shown in figures 19, 20, 22 and 23. ID 1.7-1.14 have been tested on male catheters and 1.15-1.22 were tested on female catheters.

The catheters tested as ID 1.7-1.13 and 1.15-1.21 were Polyurethane catheters of the type marketed by Coloplast A/S under the brand name "SpeediCath ®"-catheters, whereas the prior art catheter (ID 1.14 and ID 1.22) tested were PVC grade catheters marketed by Hollister Inc under the brand name "VaPro®"-catheters. All types of catheters were of size CH12. In the SpeediCath® catheters (ID 1.7-1.13 and ID 1.15-1.22) only one drainage opening was made by laser cutting and in the catheters of ID 1.14 and ID 1.22, one of the two existing drainage openings was blocked prior to testing, as described above.

It is preferable, if the suction pressure at all times is below the pressure reached inside a normal functioning bladder. And in particular, suction pressures of approximately half of the level of a prior art catheter is an improvement. Thus, embodiments relate to an intermittent urinary catheter having drainage openings and being configured for providing a pressure pulse below a threshold value of 350 mBar when tested as described in Example 1, with the modification that the immersion depth is 50cm and the height difference between the drainage opening and the outlet is 25 cm. Further embodiments relate to an intermittent urinary catheter having drainage openings and being configured for providing a pressure pulse below a threshold value of 300 mBar when tested as described in Example 1, with the modification that the immersion depth is 50cm and the height difference between the drainage opening and the outlet is 6 cm. Related embodiments relate to an intermittent urinary catheter having drainage openings and being configured for providing a pressure pulse below a threshold value of 200 mBar. Related embodiments relate to an intermittent urinary catheter having drainage openings and being configured for providing a pressure pulse below a threshold value of 100 mBar.

**Example 3:**

Another test was performed to evaluate the number of drainage openings needed for providing an optimal flow rate through an intermittent catheter according to the disclosure. In this test, 108 prototype catheters were made and the flow through each catheter was determined. The 108 catheters were in three CH sizes, CH10, CH12 and CH16. The catheters were provided with drainage openings of three sizes, a diameter of 0.4 mm, a diameter of 0.6 mm and a diameter of 0.8 mm. The number of drainage openings were varied between 15 and 240 as was the positioning of the drainage openings in rows, which was varied between 3 and 6 rows.

The results are illustrated in Table 4 below as well as in figure 27.

**Table 4**

| Size of catheter | Cross-sectional area of inside lumen (mm$^2$) | Sum of cross-sectional area of drainage openings needed for flow-convergence (mm2) |
|---|---|---|
| CH10 | 3.98 | 4 |
| CH12 | 5.52 | 5.5 |
| CH16 | 11.05 | 11 |

The results indicate, that when the total sum of the cross-sectional area of the drainage openings (the total inflow area) reaches the level of the cross-sectional area of the inner lumen of the catheter, then the flow-rate through the catheter does not increase any further. In other words, the flow converges when the total inflow area reaches the level of the cross-sectional area of the inner lumen.

**Example 4:**

In the following various examples of number of drainage openings and size of drainage openings are given based on different CH-sizes of the intermittent urinary catheter.

In these examples, the aim is to ensure complete filling of the inside lumen of the tubular portion of the catheter, meaning that the total inflow area of the drainage openings exceeds the cross-sectional area of the inside lumen of the tubular portion just distally of the most distal drainage opening (as described above).

CH12 intermittent urinary catheters may have a diameter of 2.65 mm of the inside lumen corresponding to a cross-sectional area of 5.5mm$^2$ provided that the cross-section of the lumen is circular. If an intermittent urinary catheter having drainage openings with a cross-sectional area of 0.4mm$^2$ is used, then the number of drainage openings should exceed 14 to ensure complete filling of the inside lumen. This means that for a CH12 catheter with a cross-sectional area of 5.5 mm$^2$, and provided with drainage openings with an area of 0.4 mm$^2$, the first predetermined number of drainage openings is 14. If the drainage openings are smaller, such as having a diameter of 0.4 mm corresponding to an area of 0.12 mm$^2$, then a first predetermined number of drainage openings is 46. Further examples are given in the table below:

**Table 5**

| Catheter size - CH | Diameter of inside lumen | Diameter of drainage opening (mm) | Number of drainage openings |
|---|---|---|---|
| 10 | 2.25 | 0.2 | 128 |
| | | 0.4 | 32 |
| | | 0.6 | 15 |
| | | 0.7 | 11 |
| 12 | 2.65 | 0.2 | 176 |
| | | 0.4 | 44 |
| | | 0.6 | 20 |
| | | 0.7 | 15 |

(continued)

| Catheter size - CH | Diameter of inside lumen | Diameter of drainage opening (mm) | Number of drainage openings |
|---|---|---|---|
| 14 | 3.22 | 0.2 | 261 |
| | | 0.4 | 66 |
| | | 0.6 | 29 |
| | | 0.7 | 22 |
| 16 | 3.75 | 0.2 | 351 |
| | | 0.4 | 88 |
| | | 0.6 | 39 |
| | | 0.7 | 29 |

### Example 5:

A number of catheters were tested in a model of a bladder - a porcine bladder. The bladder was filled with 200 ml of water and drained by a catheter inserted into a urethral pathway into the bladder. In all tests, the catheters were inserted so that the most distal drainage opening was positioned one cm above the bladder neck.

For a first series of tests, various prototypes were made. The variations in the prototypes relate to the number of drainage openings (12, 24 or 48), the positioning of the drainage openings in 2, 3 and 4 rows around the catheter, i.e. with 180 degrees, 120 degrees and 90 degrees between them, respectively, the positioning of the drainage openings in the longitudinal direction so that they were positioned 1.4 mm from each other in one type and 2.0 mm from each other in a second type and finally the size of the drainage openings was varied so that some of the catheters were made with drainage openings with a diameter of 0.4 and some were made with drainage openings with a diameter of 0.8 mm. This means that for example, one catheter was a catheter having 12 drainage openings positioned in 2 rows, 1.4 mm from each and having a size of 0.4 mm. Another catheter had 24 drainage openings positioned in 2 rows, 2.0 mm from each other and having a size of 0.8 mm. And so forth until 36 combinations were made. The table below shows an overview of the prototypes and the numbering used.

**Table 6**

| Number of drainage openings | Diameter of drainage openings | rows | Longitudinal distance | Prototype number |
|---|---|---|---|---|
| 12 | 0.4 | 2 | 1.4 | 15 |
| | | | 2.0 | 22 |
| | | 3 | 1.4 | 35 |
| | | | 2.0 | 29 |
| | | 4 | 1.4 | 8 |
| | | | 2.0 | 28 |
| | 0.8 | 2 | 1.4 | 21 |
| | | | 2.0 | 18 |
| | | 3 | 1.4 | 14 |
| | | | 2.0 | 36 |
| | | 4 | 1.4 | 31 |
| | | | 2.0 | 10 |

(continued)

| Number of drainage openings | Diameter of drainage openings | rows | Longitudinal distance | Prototype number |
|---|---|---|---|---|
| 24 | 0.4 | 2 | 1.4 | 30 |
| | | | 2.0 | 16 |
| | | 3 | 1.4 | 11 |
| | | | 2.0 | 4 |
| | | 4 | 1.4 | 5 |
| | | | 2.0 | 19 |
| | 0.8 | 2 | 1.4 | 9 |
| | | | 2.0 | 27 |
| | | 3 | 1.4 | 1 |
| | | | 2.0 | 33 |
| | | 4 | 1.4 | 26 |
| | | | 2.0 | 12 |
| 48 | 0.4 | 2 | 1.4 | 23 |
| | | | 2.0 | 7 |
| | | 3 | 1.4 | 6 |
| | | | 2.0 | 17 |
| | | 4 | 1.4 | 32 |
| | | | 2.0 | 24 |
| | 0.8 | 2 | 1.4 | 25 |
| | | | 2.0 | 34 |
| | | 3 | 1.4 | 3 |
| | | | 2.0 | 13 |
| | | 4 | 1.4 | 20 |
| | | | 2.0 | 2 |

All of these 36 catheters were tested in the bladder-model and the results were compared to testing of a prior art catheter having only two drainage openings.

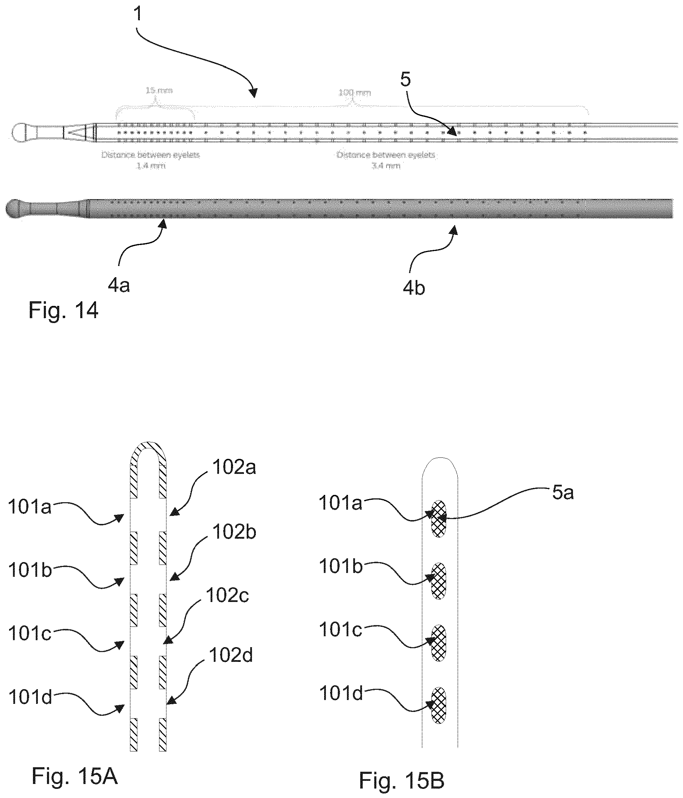

In a second test series, a catheter with 144 drainage openings of a diameter of 0.4 mm were used. In this catheter, the drainage openings were positioned so that there was a drainage zone of 15 mm having 44 drainage openings positioned in four rows with 1.4 mm between them in a longitudinal direction. The catheter is illustrated in figure 14.

In the first series of tests, each catheter was inserted 10 times, so the bladder was emptied 10 times by the same catheter. During each emptying, if the flow completely stops (i.e. clogging occurs), the catheter was repositioned slightly by rotating it approximately 45 degrees. This was repeated until all of the liquid in the bladder was drained into the catheter.

In the second test series each catheter was inserted once followed by another catheter and so forth. This round was then repeated 10 times. Again, if clogging occurred, the catheter was rotated 45 degrees and this was repeated until the bladder was empty.

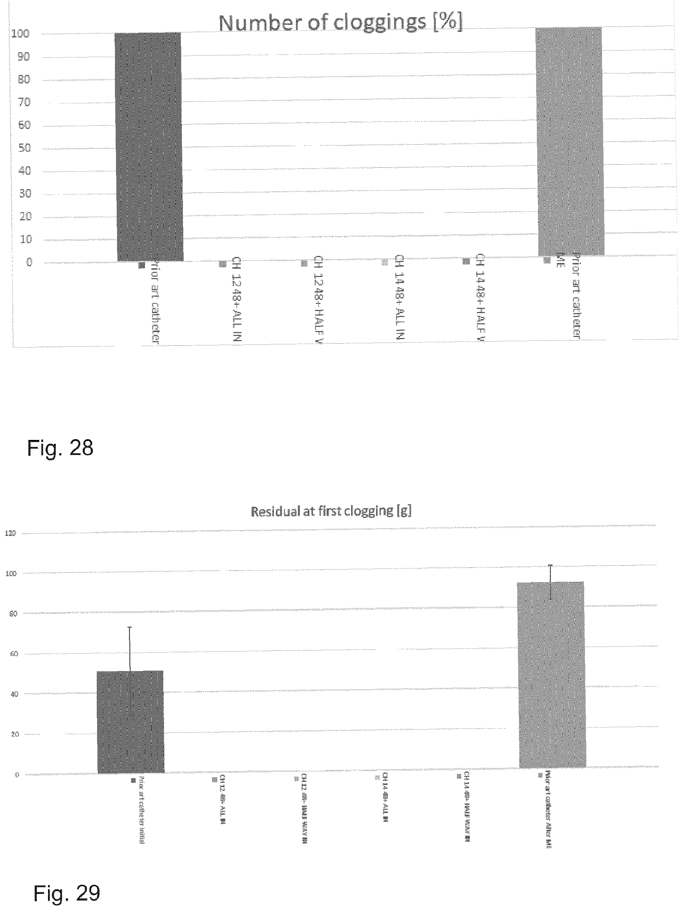

The test results are illustrated in figures 28 to 36, and will be summarised below.

An overall conclusion is that no clogging occurs in an intermittent urinary catheter with multiple small drainage openings, i.e. in none of the prototypes mentioned above. The flow may be reduced during the test but will continue by itself until the bladder is empty.

Since all of the prototypes has continuous flow until the bladder is completely drained, none of the prototypes leaves any residual urine.

For the prior art catheters (VaPro® and LoFric®) the results are that there is more often than not a need for repositioning before the bladder can be completely emptied. Clogging as defined as a complete flow-stop always occur. This means that if the catheter was removed after the first flow stop, residual urine would in most cases be left in the bladder.

*Example 6:*

The aim of this first simulation is to simulate the flow and pressure and the resulting drag-forces in the bladder during voiding. Flow rates of 2 ml/s and 10 ml/s are used, since this is a typically range of flow rates for draining the bladder with an intermittent catheter. Several cases were simulated as mentioned here and illustrated in Table 7.

Case no. 1: A free catheter with drainage openings open on two sides. Free means that there is neither any blockage of the drainage openings, nor is there any wall close to the drainage openings. This simulates the situation, when the catheter is situated in the bladder and the bladder-wall is sufficiently far from the catheter so that it does not influence the flow into the catheter.

Case no. 2: A catheter having only one side of drainage openings facing a wall positioned 5 mm from the catheter surface. This simulates the situation, where drainage openings are only open on one side of the catheter, the other side(s) being potentially blocked by bladder wall tissue. The open drainage openings face a wall situated 5 mm from the drainage openings simulating the situation, where the bladder wall is close to the drainage openings and thus influences and is influenced by the inflow into the catheter.

Case no. 3: Same as in case no. 2 - except the wall is positioned 1 mm from the catheter surface. This simulates the situation, where the bladder wall is close to the drainage openings and influences and is influenced by the inflow into the catheter.

Case no. 4: A catheter having two open sides of drainage openings and where one side of the drainage openings face a wall positioned 1 mm from the catheter surface. This simulates the situation, where the catheter is positioned in the bladder and the inflow into the catheter on one side of the drainage openings influences and is influenced by the bladder wall, whereas the other side of the drainage openings are un-influenced by the bladder wall, because the bladder wall is further away from the catheter.

Case no. 5: A catheter having two open sides of drainage openings, and where both sides of the drainage openings are facing a wall positioned 1 mm from the catheter surface. This simulates the situation, where the inflow into the catheter on both sides is influenced by and influences the bladder wall.

These 5 cases are combined with three sets of drainage openings.

Configuration 1: Two eyelets positioned one on either side of the catheter with 20mm from top of the lower eyelet to bottom of the higher eyelet. Eyelet size is 2.5 mm$^2$ corresponding to size of eyelet in a prior art catheter Configuration 2: 12 drainage openings positioned 6 on either side of the catheter with 3 mm between them (centre to centre). Size of drainage openings is a diameter of 1 mm.

Configuration 3: 24 drainage openings positioned 12 on either side of the catheter with 3 mm between them (centre to centre). Size of drainage openings is a diameter of 0.4 mm. In these configurations, the flow is simulated as half of the aim so as to simulate 48 drainage openings.

Table 7 below indicates the different combinations, which were used in the simulations.

**Table 7**

| Configuration of drainage openings | Case 1 | Case 2 | Case 3 | Case 4 | Case 5 |
|---|---|---|---|---|---|
| 1 | Comb. 1.1 | Comb. 1.2 | Comb. 1.3 | Comb. 1.4 | Comb. 1.5 |
| 2 | Comb. 2.1 | Comb. 2.2 | Comb. 2.3 | Comb. 2.4 | Comb. 2.5 |
| 3 | Comb. 3.1 | Comb. 3.2 | Comb. 3.3 | Comb. 3.4 | Comb. 3.5 |

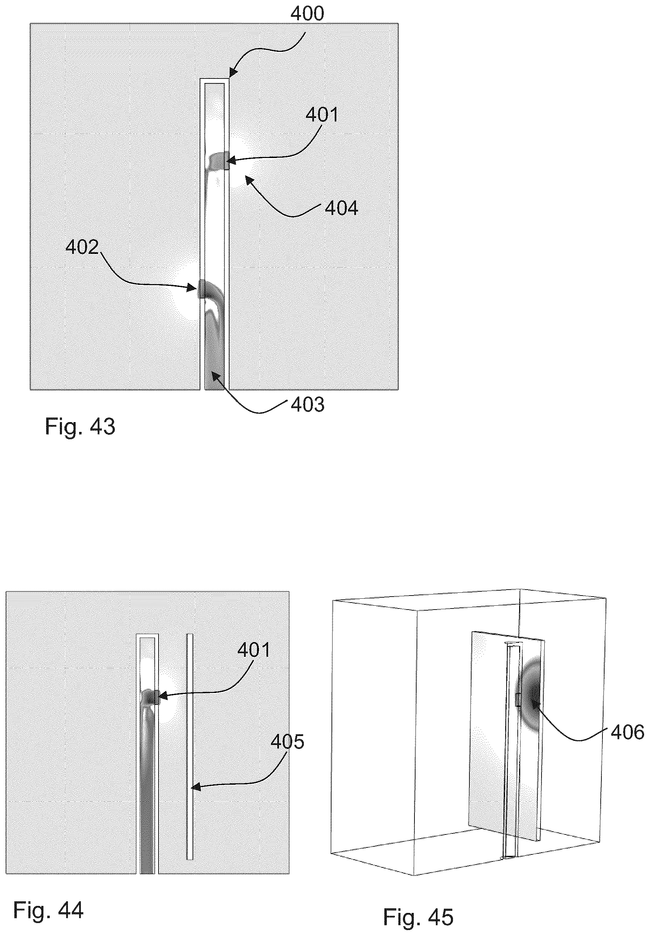

The results are shown in Figures 43 to 61 and will be summarised below.

The results show that an intermittent urinary catheter as described above, having multiple small drainage openings, will provide a reduced drag-force (or suction-force) compared to an intermittent catheter having two larger eyelets. The drag-force is the influence on an object (e.g. a bladder-wall) positioned close to the catheter, when the catheter is draining urine.

The drag-force in mN for case 2, 3, 4 and 5 are summarised in Table 8 below, which illustrates the impact the different configurations of drainage openings/eyelets have on an object positioned close to the intermittent catheter. Nominative values are shown, meaning that we have indicated all values as positive, even if the force is actually a dragging force and hence negative. The force is shown for two different flow rates, which are the normal limits for flow rates through an intermittent urinary catheter.

**Table 8**

| Case: | Case 2 | | Case 3 | | Case 4 | | Case 5 | |
|---|---|---|---|---|---|---|---|---|
| Flow rate | 2ml/s | 10ml/s | 2ml/s | 10ml/s | 2ml/s | 10ml/s | 2ml/s | 10ml/s |
| 1 | 0.005 | 0.089 | 0.141 | 2.840 | 0.028 | 0.517 | 0.069 0.042 | 1.221 0.714 |
| 2 | 0.003 | 0.059 | 0.056 | 0.999 | 0.020 | 0.321 | 0.036 | 0.540 |
| 3 | 0.001 | 0.011 | 0.013 | 0.176 | 0.005 | 0.058 | 0.012 | 0.123 |

The drag-force for combination 1.3, is by far the largest. This is 2.840 mN at a flow-rate of 10 ml/s. This is a prior art catheter having two eyelets, where one is blocked and the other one positioned 1 mm from an object. In that case, the influence on the object (which may be a bladder wall during a draining procedure) will be significant. If this result is compared to the situation, where the intermittent catheter is provided with 12 smaller drainage openings (combination 2.3), then the drag-force is approximately 1/3, when one side of the drainage openings are closed and the drainage openings on the other side are 1 mm from an object. The drag-force is even further reduced if a catheter having 48 drainage openings are used - then the drag-force is less than 1/10th of the drag-force for a prior art catheter.

Similarly, simulating an object close to both sides of the catheter (case 5) shows that the drag-force is significantly reduced for an intermittent urinary catheter having multiple small drainage openings compared to a prior art catheter. The table shows two values for the prior art catheter, indicating that the eyelet positioned lowest (the left, shown first as the first value in the table) dominates the flow as mentioned earlier. This means that most of the flow-rate will be through this eyelet. A comparison for the flow rate of 10 ml/s shows that for the highest influencing eyelet, the drag-force is reduced by one half, when 12 drainage openings are used and reduced to approximately 1/10th when 48 drainage openings are used.

In a second series of simulations, the flow and pressure circumferentially around the catheter were simulated. The catheter was simulated as being a CH12 catheter with drainage openings positioned in two rows, 180 degrees apart from each other, meaning that they were positioned directly opposite from each other.

Flow and pressure was simulated in three positions circumferentially around the catheter:

Case 6: In alignment with the drainage openings (at 0 degrees),

Case 7: At 45 degrees displaced from the drainage openings around the external surface of the catheter

Case 8: At 90 degrees displaced from the drainage openings around the external surface of the catheter.

Again, three different catheters were used for the simulation:

Configuration 1: Two eyelets positioned one on either side of the catheter with 20mm from top of the lower eyelet to bottom of the higher eyelet. Eyelet size is 2.5 mm$^2$ corresponding to size of eyelet in a prior art catheter

Configuration 2: 12 drainage openings positioned 6 on either side of the catheter with 3 mm between them (centre to centre). Size of drainage openings is a diameter of 1 mm.

Configuration 3: 24 drainage openings positioned 12 on either side of the catheter with 3 mm between them (centre to centre). Size of drainage openings is a diameter of 0.4 mm. In these configurations, the flow is simulated as half of the aim so as to simulate 48 drainage openings.

The results are shown in Figures 65 to 69, 71 to 73 and 75 to 77 and will be summarised below.

The simulated pressure at the three different positions and for the different configurations of drainage openings are shown in the table below. The pressure is a relative pressure at 1 mm from the catheter surface, and the pressure is relative to a pressure of zero in the bladder.

**Table 9**

| Case | 6 | 7 | 8 |
|---|---|---|---|
| Conf. of drainage openings | | | |
| 1 - upper | 45 | 6 | <1 |
| 1 - lower | 100 | 12 | 1 |
| 2 | 14 | 1 | 0.2 |
| 3 | 0.7 | 0.05 | 0.001 |

The results in Table 9 clearly shows that the pressure as a result of the draining through the drainage openings are markedly higher for the configuration with only two large drainage openings. It is more than a factor 100 compared to the results on a catheter having 48 small drainage openings.

The results further show that the pressure at 90 degrees (case 8 above), is close to zero for all catheter configurations. This means that if further rows were added at this position, then it is possible to distribute the pressure around the circumference much more evenly than for a catheter having only two rows opposite each other. This would further reduce the pressure at each drainage opening.

***Example 7: Testing of pressure profile in the bladder.***

The drag-force on an object resulting from influence from suction by the drainage openings and the suction pressure-profile in the bladder during the voiding phase may be tested as follows - see figure 62 and 63. In one measuring configuration a catheter is positioned in a container, which simulates urine in the bladder. The container needs to have a volume significant higher than what is displaced by the catheter. As an example for a CH 12 catheter with 50 mm inserted into the container, the volume of the container should be in range of 100-400 ml. In order to prolong the period of measuring, the container may be equipped with a pump, that recycles what is drained through the catheter. The distance from drainage openings to any wall of the container should exceed 10 mm, in order to limit the influence of the container wall with respect to pressure profile.

A holding device holding a plate and a load cell or a pressure sensor depending on what is measured, is provided, and arranged to 'scan' the drag-force/pressure of the flow-zone around the catheter. In order to disturb the flow minimally, the pressure sensor should be held in position by e.g. a very thin rod, extending radially with respect to the catheter.

A scanner motor allows the load cell/sensor to be moved around the catheter during measuring. Either the motor or actuator scans the catheter in circular movements followed by longitudinal displacement, or the motor/actuator moves the sensor in a helical movement around the catheter. It is preferred that the distance to the catheter is kept as precise as possible.

During emptying of the catheter, the data is logged in given intervals, which provides a 'grid' of drag-force/pressure in the flow at various distances.

Instead of rotating the sensor around the catheter, an option is to rotate the catheter slowly during measuring. This simplifies the motor arrangement significant. In this case a rotational sealing and motor is arranged at the lower part of container.

In order to ensure that the catheter is held straight during measuring - and to ensure that the distance to the sensor is as constant as possible, the catheter may be fixed rotationally at the tip.

**Detailed Description of the Drawing**

Embodiments, and features of the various exemplary embodiments described in this application, may be combined with each other ("mixed and matched"), unless specifically noted otherwise.

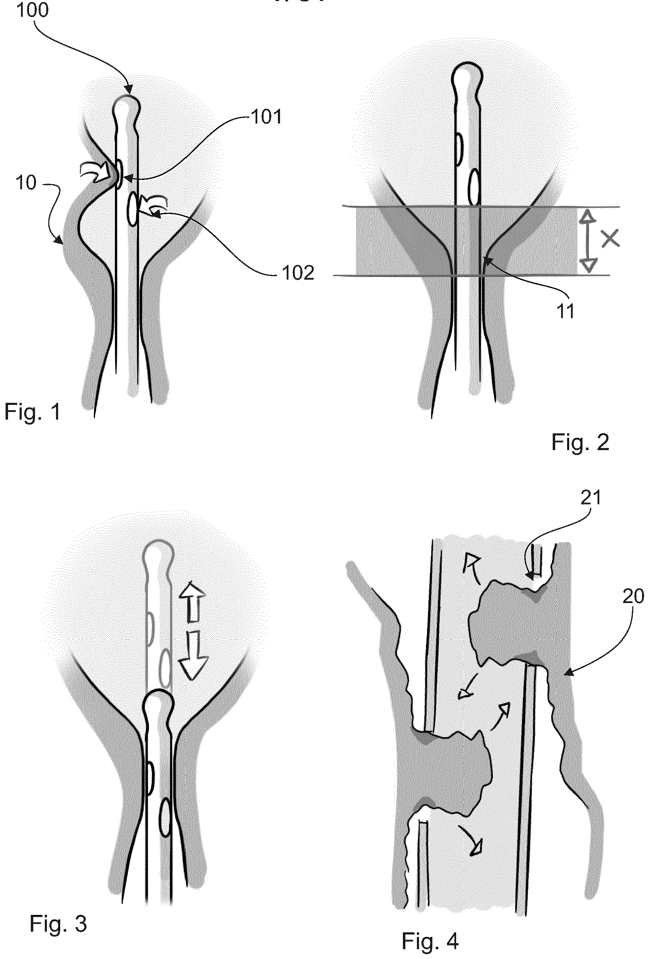

Figures 1-4 illustrate various issues with prior art catheters. Figure 1 illustrates a part of a prior art catheter **100** having two drainage openings **101, 102** inserted into the bladder **10**. During catheterisation, one drainage opening **101** may be blocked by the bladder wall tissue as illustrated, and then all draining of urine from the bladder occurs through the second drainage opening **102**. This situation creates a high suction effect through the second drainage opening **102,** which may cause bladder wall tissue to enter into contact with this second drainage opening **102,** as described above. Figure 2 illustrates a part of a prior art catheter **100** situated in the bladder **10**. This figure illustrates a situation in which the prior art catheter sits too high in the bladder **10,** i.e. above the bladder neck **11** and thus the bladder **10** will not empty completely during catheterisation. Residual urine in the bladder may lead to urinary tract infection. Figure 3 illustrates how the prior art catheter **100** then has to be moved up and down to try to alleviate pooling of residual urine. However, this moving up and down of the catheter may lead to the situation illustrated in figure 4, namely that urethral tissue **21** from the bladder **10** or the upper urethra **20** enters into the drainage openings and thus be subjected to chafing during the movement of the catheter up and down.

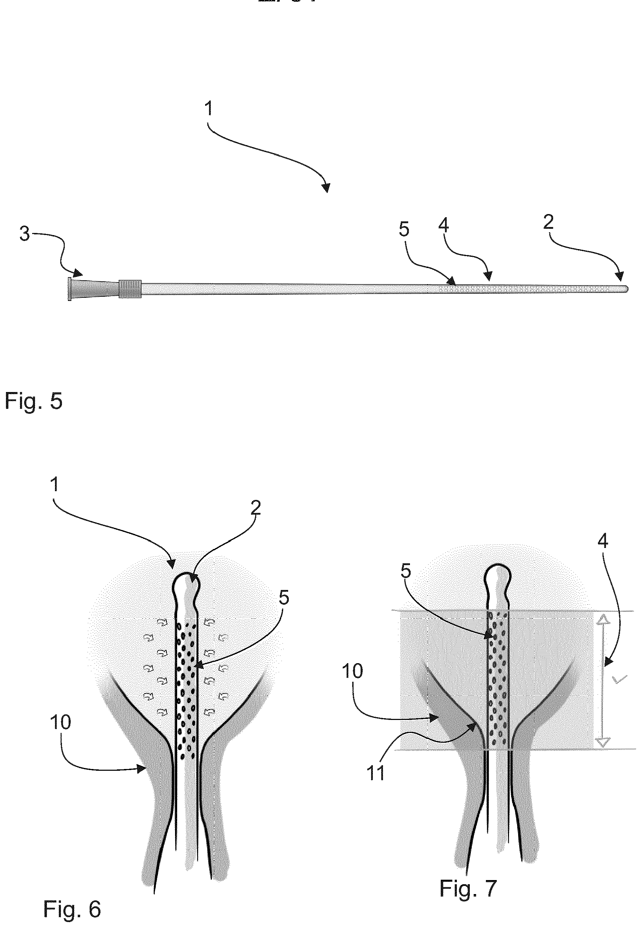

Figure 5 illustrates one embodiment of an intermittent urinary catheter **1** as described herein. The intermittent urinary catheter is provided with a closed tip portion **2** in a proximal insertion end, in figure 5 the closed tip portion illustrated is a Nelaton tip. The intermittent urinary catheter **1** is further provided with a connector **3** in a distal outlet end. Multiple drainage openings **5** are positioned in a drainage portion **4** of the intermittent catheter. In the illustrated embodiment, multiple drainage openings **5** are positioned in four rows positioned in pairs with 180 degrees between the pairs. Only the two rows on one side of the intermittent urinary catheter **1** are visible in figure 5.

Figures 6 and 7 illustrate embodiments of an intermittent urinary catheter **1** as described herein positioned with the drainage portion 4 extending into the bladder **10**. In this embodiment, the closed tip **2** is a flex-tip. In figure 6, multiple drainage openings **5** are positioned scattered across the surface of the intermittent catheter **1**. The Figure 6 embodiment illustrates how a plurality of drainage openings **5** allows for inflow of urine in multiple positions along the drainage portion **4** of the intermittent catheter. Furthermore, having multiple drainage openings **5** reduces the possibility of sucking the bladder wall tissue into a drainage opening **5** during catheterisation, as described above. Figure 7 illustrates how the bladder **10** can be completely emptied by the intermittent catheter **1** having multiple drainage openings **5**. This is because the likelihood of all of the multiple drainage openings **5** being blocked simultaneously is very small; therefore, urine will continue to drain until the bladder **10** is empty. Furthermore, in the embodiments of figures 6 and 7, the drainage portion **4** is relatively long compared to prior art catheters, thus allowing for presence of drainage openings **5** also at the bladder neck **11,** thereby helping to ensure emptying of the bladder **10**.

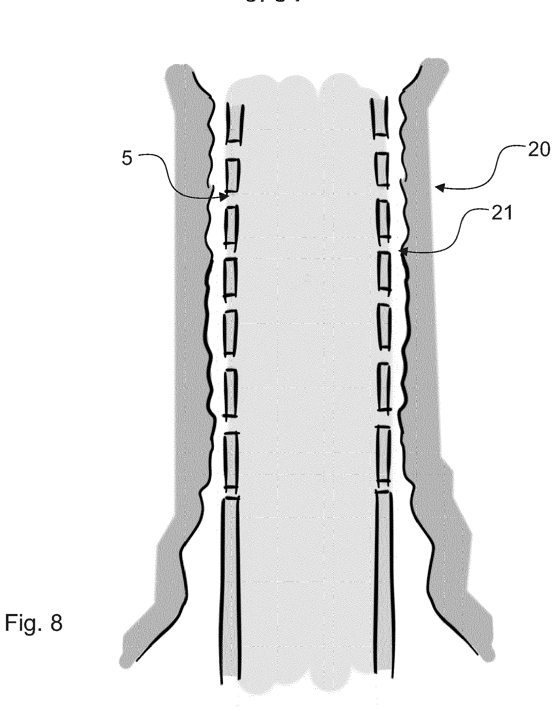

Figure 8 illustrates a portion of one embodiment of an intermittent urinary catheter **1** as described herein positioned in the upper part of the urethra **20**. The figure illustrates that the tissue **21** of the urethra does not enter in through the multiple drainage openings **5,** thereby the intermittent catheter 1 reduces the risk of influencing the urethral tissue **20**.

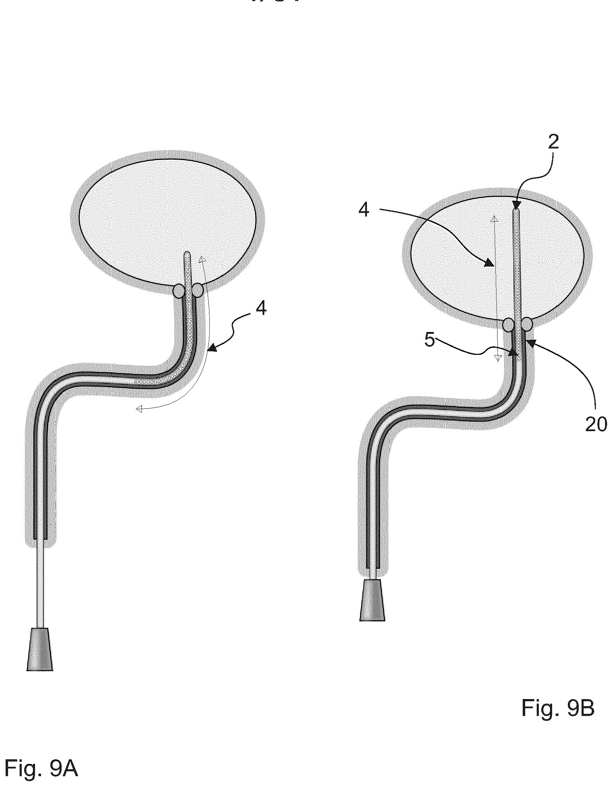

Figures 9A and 9B are schematic drawings illustrating that the drainage portion **4** of embodiments of a urinary catheter as described herein can be so long that even in the case the intermittent catheter 1 is inserted until the closed tip **2** reaches the top of the bladder (figure 9B), then some drainage openings **5** are positioned below the bladder neck, i.e. in the upper urethra **20**.

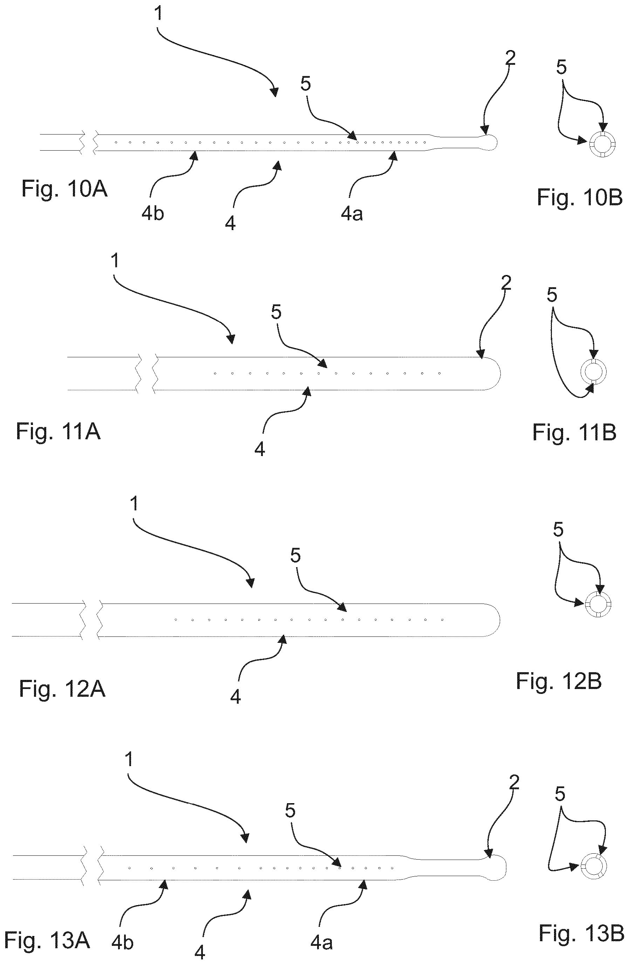

Figures 10A, 10B illustrate an embodiment of an intermittent catheter **1** of this disclosure. In the embodiment, the catheter has 108 drainage openings **5** positioned with 27 in each row in the longitudinal direction and with 90 degrees between them circumferentially around the catheter surface - see figure 10B illustrating a cross-sectional view. The drainage openings **5** are positioned such that, starting from a proximal insertion end, the 44 most proximal ones are positioned with 11 mm between them in the longitudinal direction, whereas the 64 distal ones are positioned with 22 mm between them in the longitudinal direction. Thus, the drainage portion **4** is in this embodiment divided into a first drainage portion **4a** and a second drainage portion **4b.** In figure 10A only one longitudinal row is illustrated.

In figure 10A, the embodiment includes a first degree of perforation in a portion of the catheter and a second degree of perforation in a more distal portion of the catheter. This corresponds to a first degree of perforation in a first drainage portion **4a** and a second degree of perforation in a second drainage portion **4b.**

The tip portion **2** is illustrated as being a flex-tip. Only part of the longitudinal extent of the intermittent urinary catheter is illustrated in figure 10A. The catheter illustrated in figures 10A, 10B will typically be around 40 cm long and will be useful as a male catheter.

Figures 11A and 11B illustrates another embodiment of an intermittent catheter **1,** where the drainage openings **5** are positioned in the drainage portion **4** in two longitudinal rows, and 14 drainage openings in the longitudinal direction 2.1 mm apart from each other, meaning a total of 28 drainage openings. However, the catheter could also be provided with four longitudinal rows so that it would be provided with a total of 56 drainage openings. Only one longitudinal row is illustrated in figure 11A.

The tip portion **2** is illustrated as being a Nelaton-tip. Only part of the longitudinal extent of the catheter is illustrated in figure 11 A, the catheter will typically be approximately 15 to 20 cm long, such as approximately 17 cm. The intermittent urinary catheter in figures 11A and 11B will be useful as a female catheter.

Figures 12A, 12B illustrate another embodiment of an intermittent urinary catheter **1,** where the drainage portion **4** include drainage openings **5** positioned in four longitudinal rows. The drainage openings **5** are positioned with 16 drainage openings in the longitudinal direction, 2.1 mm apart from each other, meaning a total of 68 drainage openings. Only one longitudinal row is illustrated. The tip portion **2** is illustrated as being a Nelaton-tip. Only part of the longitudinal extent of the catheter is illustrated in figure 12 A, the catheter will typically be approximately 15 to 20 cm long, such as approximately 17 cm. The intermittent urinary catheter in figures 12A and 12B will be useful as a female catheter.

Figures 13A, 13B illustrate another embodiment of an intermittent urinary catheter **1,** where the drainage portion **4** include a first drainage portion **4a** and a second drainage portion **4b.** The drainage openings **5** are positioned in three longitudinal rows, with a first and second degree of perforation. The first proximal section, corresponding to the first drainage portion **4a** has 16 drainage openings in the longitudinal direction, 2.1 mm apart from each other, i.e. a proximal section with 48 drainage openings. The second more distal section, corresponding to the second drainage portion **4b** has 6 drainage openings in the longitudinal direction, 3.5 mm apart from each other, i.e. a distal section with 18 drainage openings. A total number of 64 drainage openings. The tip portion **2** is illustrated as being a flex-tip. Only part of the longitudinal extent of the intermittent urinary catheter is illustrated in figure 13A. The catheter illustrated in figures 13A, 13B will typically be around 40 cm long and will be useful as a male catheter.

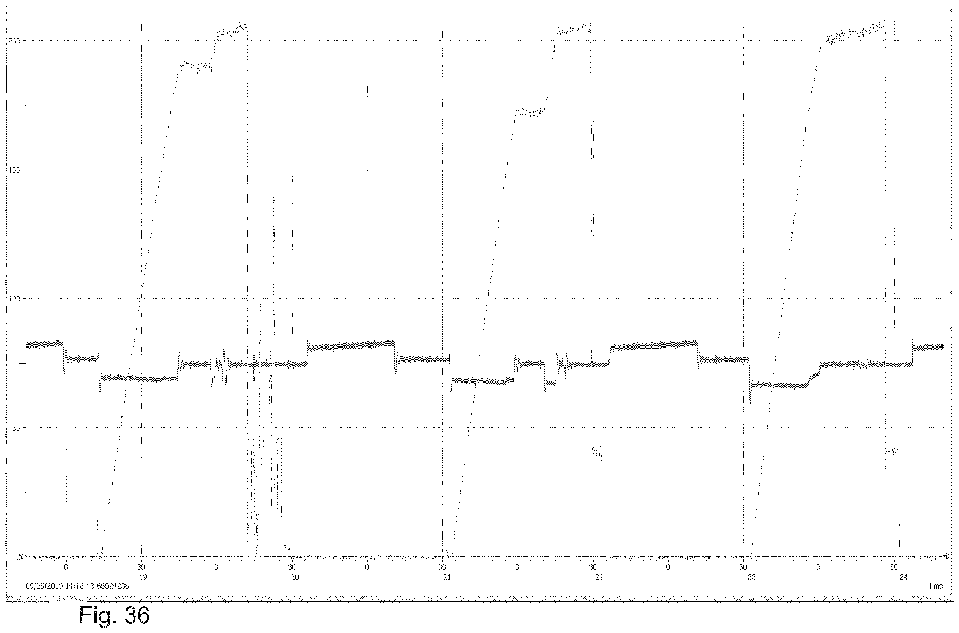

Figure 14 illustrates another embodiment of an intermittent urinary catheter **1,** having a total number of drainage openings **5** of 144. These drainage openings **5** are also positioned in two portions with different degrees of perforation, a first drainage portion **4a** having 44 drainage openings positioned in four longitudinal rows and with a distance of 1.4 mm between them and a second drainage portion **4b** having 100 drainage openings positioned in four longitudinal rows and with a distance of 3.4 mm between them. The figure illustrates a cross sectional view as well as a side view. This prototype catheter with 144 drainage openings was used in the tests shown in figure 36 and 30 and 31. The catheter is a male catheter with a standard size of CH12 but it has a drainage portion extending 10 cm in the longitudinal direction, the drainage portion having two different degrees of perforations. In the first drainage portion, the degree of perforation is 0.12 and in the second drainage portion, the degree of perforation is 0.06.

Figures 15a, 15B illustrate another embodiment, where larger inlet holes **101a, 101b, 101c, 101d, 102a, 102b, 102c, 102d,** are provided with a mesh structure **5a.** In this embodiment, the mesh structure **5a** assists in providing small drainage openings, thus the size of the inlet holes influences the flow in the catheter to a lesser degree.

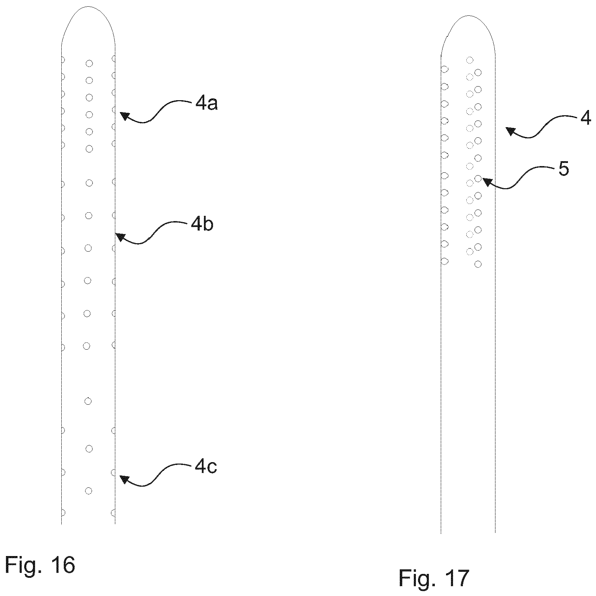

Figure 16 illustrates a side view of an embodiment of an intermittent urinary catheter **1** having three different degrees of perforation, a first degree of perforation in a proximal drainage portion **4a,** a second degree of perforation in a more distal section (a second drainage portion **4b**) and a third degree of perforation in a third drainage portion **4b** distally thereof.

Figure 17 illustrates a side view an embodiment of an intermittent urinary catheter having a drainage portion **4** with drainage openings **5** positioned in three rows. Two rows are visible from in the view, but the third row is positioned on the rear side of the catheter and thus illustrated in ghost lines.

Figure 18 illustrates schematically a pressure pulse occurring in an intermittent catheter during emptying of the bladder. The figure illustrates the pressure-difference as a function of time during a series of cloggings of the drainage openings in a catheter. The pressure pulse occurs as a sudden decrease in pressure over a very short period of time - in the order of 100 milliseconds or less. It is illustrated as the peaks on the curves in the figure. As explained above, the pressure pulse occurs because the movement of the urine through the catheter is abruptly stopped due to tissue blocking the drainage openings.

Figures 18 to 23 illustrate results obtained by testing various catheters using the test set-up in figure 24-26. Figure 18 illustrates results from testing of male catheters with a drainage height of 15-20 cm and under a level of water of 10 cm $H_2O$. Starting from the left in figure 18, this graph illustrates the pressure pulse obtained inside a prior art catheter of size CH16 having two regular drainage openings with a largest dimension of 5.6 mm. One of the drainage openings were closed off prior to testing. From figure 18, it can be seen that the pressure pulse exceeds 200 mBar. Going towards the right of the figure, the next graph illustrates the pressure pulse obtained inside a prior art catheter of size CH 12 having two drainage openings with a largest dimension of 3.9 mm. Such a catheter provides a pressure pulse of around 200 mBar. The third graph from the left illustrates the pressure pulse obtained on a prior art catheter of CH10, having drainage openings of a largest dimension of 3.4 mm. Here the pressure pulse exceeds 100 mBar. The fourth graph from the left illustrates the pressure pulse obtained on an intermittent urinary catheter as described herein and having one open drainage opening with a largest dimension of 1 mm. The graph illustrates that the pressure pulse only reaches to around 40 mBar. The graph towards the far right illustrates the pressure pulse for an intermittent urinary catheter as described herein and having one open drainage opening with a largest dimension of around 0.4 mm. Here the pressure pulse is virtually non-existent - there is almost no peak on the curve.

Figure 19 illustrates results from testing of male catheters with a drainage height of 25 cm and under a level of water of 50 cm H2O. Starting from the left in figure 19, the graph illustrates the pressure pulses obtained in catheters with one single open drainage opening and with the single drainage opening increasing from the left in the figure towards the right. The results are also reported in Table 2 below. It can be seen that for a drainage opening of 4 mm in largest dimension, the pressure pulse (under these test-conditions) reached 652 mBar, whereas towards the left, the pressure pulse (under these test-conditions) is as low as 15 mBar when the drainage opening is 0.19mm. Levels of less than 100 mBar is obtained with drainage openings smaller than approximately 0.4 mm, levels of less than 200 mBar is obtained with drainage openings smaller than approximately 0.6 mm and levels of less than 350 mBar is obtained with drainage openings smaller than approximately 1.00 mm.

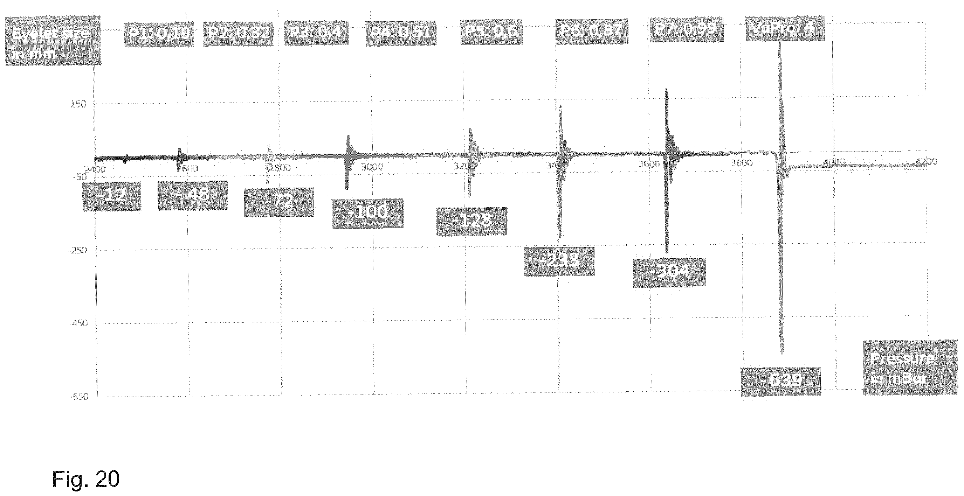

Figure 20 illustrates results from testing of female catheters with a drainage height of 6 cm and under a level of water of 50 cm H2O. Starting from the left in Figure 20, the graph illustrates the pressure pulses obtained in catheters with one single open drainage opening and with the single drainage opening increasing from the left in the figure towards the right. The results are also reported in Table 3 above. It can be seen that for a drainage opening of 4 mm in largest dimension, the pressure pulse (under these test-conditions) reached 639 mBar, whereas towards the left, the pressure pulse (under these test-conditions) is as low as 12 mBar when the drainage opening is 0.19 mm. Levels of less than 100 mBar is obtained with drainage openings smaller than approximately 0.5 mm, levels of less than 200 mBar is obtained with drainage openings smaller than approximately 0.7 mm and levels of less than 350 mBar is obtained with drainage openings smaller than approximately 1.00 mm.

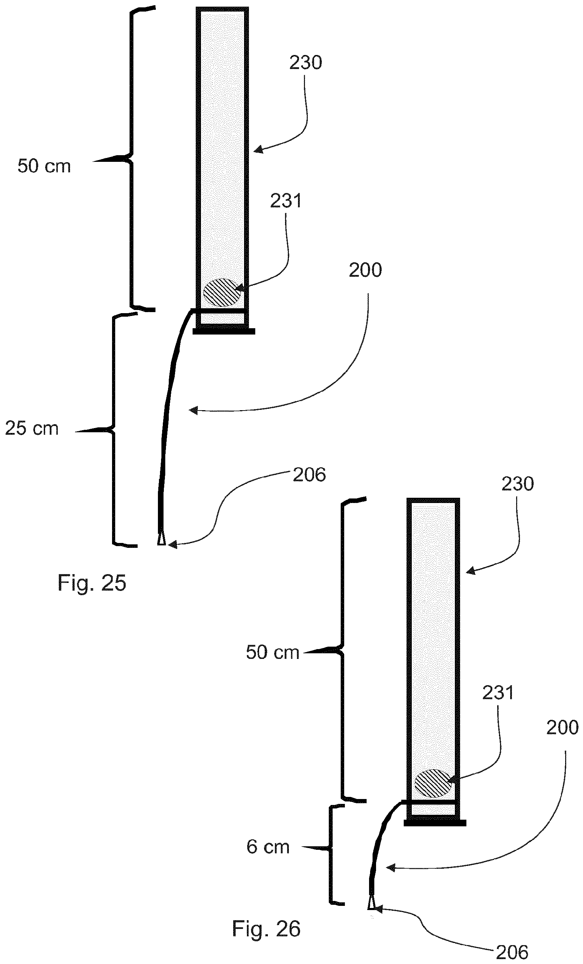

Figures 21A and 21B illustrate test results for tests performed according to the test set-up in Figures 25 and 26. Figure 21B illustrate in larger scale a correlation between how much bladder wall or urethral tissue enters into the inner lumen through the drainage openings, the size of the drainage openings and the measured pressure pulse. From the results of the tests, it is understood that a pressure pulse below 40 mBar reduces the risk of bladder wall or urethral tissue entering into the inner lumen through the small drainage openings in the intermittent catheter and reduce the risk of influence to the tissue. In embodiments of the present disclosure, an intermittent urinary catheter is achieved wherein none or very little tissue enters into the inner lumen through the small drainage openings, when the pressure pulse is below 40 mBar. A pressure pulse below 40mBar is obtained when the drainage opening has a largest dimension of below 0.7 mm. Thus, embodiments relate to an intermittent urinary catheter configured for providing a pressure pulse below 40 mBar. Related embodiments are an intermittent urinary catheter having drainage openings with a largest dimension below 0.7 mm.

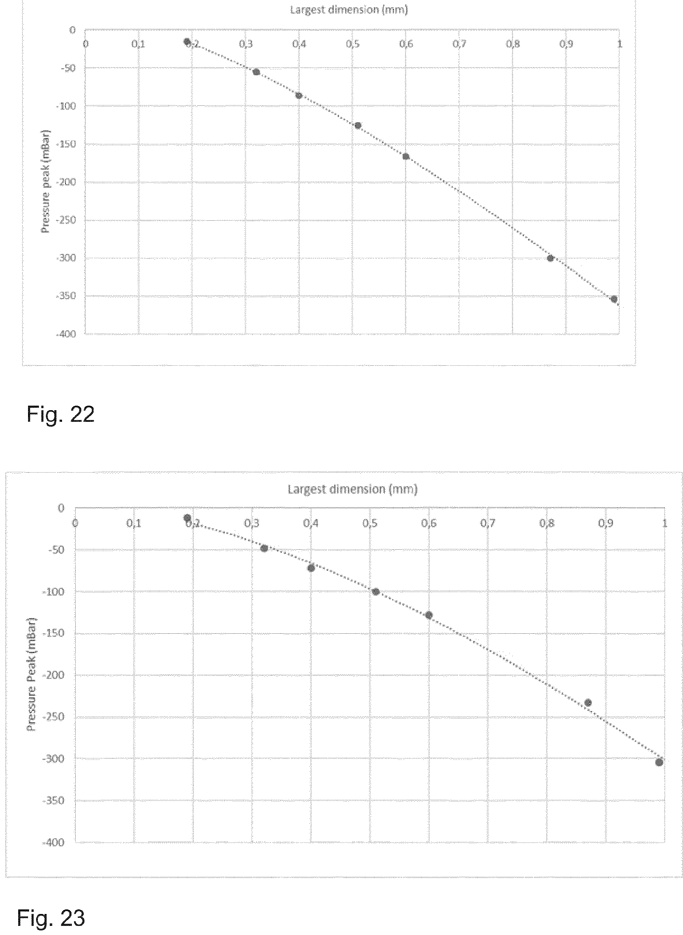

Figures 22 and 23 illustrate test results for tests performed according to the test set-up in Figures 25 and 26. The results in Figure 22 are for testing a male catheter as illustrated in Figure 25 and the results in Figure 23 are results for testing a female catheter as illustrated in Figure 27. The difference is that for the male catheter the height difference between the level of the drainage opening and the outlet of the catheter is 25 cm, wherein for the female catheter the height difference is 6 cm.

In Figures 22, and 23, the results of testing drainage openings with a largest dimension of 1 mm and below are shown. The curves illustrate that for a male catheter, the pressure pulse will be below 350 mBar, if drainage openings of less than 1 mm are used. For a female catheter, the pressure pulse will be below 300 mBar. If drainage openings of 0.8 mm are used, the pressure pulse for male will be around 260 mbar and for females around 210 mBar. If drainage openings of 0.4mm are used, then the pressure pulse for male catheter will be around 90 mBar and for female around 75 mBar.

Figure 24 illustrates a first test set-up used to measure a pressure pulse occurring inside an intermittent catheter 200 during catheterisation - and in particular, where all but a most distal one of the drainage openings have been closed. The intermittent catheter having all but the most distal drainage opening closed is immersed in a water tank 230. The drainage portion 204 of the catheter is surrounded by bladder tissue 231, illustrated by the white part floating around the drainage portion 204 of the catheter. A pressure gauge 231 connected through a wire to a pressure sensor 232 is used to measure the pressure pulse in the inner lumen of the intermittent catheter 200. The pressure sensor 232 itself is positioned close to the catheter 200 and receives input from a needle 233 inserted into the inner lumen of the catheter through the closed tip portion. The catheter exits the water tank 230 through a liquid tight connection 234. In this first test set-up, the height difference between the catheter tip and the outlet of the catheter 206 is 10-15 cm. In this first test set-up the catheter is immersed 10 cm below the surface of the water in the water tank 301.

Figure 25-26 illustrate a second and third test set-up used to measure a pressure pulse occurring inside an intermittent catheter 200 during catheterisation. Again, the catheters were tested under conditions, where all but a most distal one of the drainage openings have been closed. The only changes with respect to the test in figure 24 is the immersion depth of the catheters, which in this second and third test set-up was 50 cm, as illustrated by the higher water tank 301 in the figure. A further difference is the height difference between the catheter tip and the outlet 206. In figure 25, this height difference was 25 cm, corresponding to a typical height difference for a male user during catheterisation and in figure 26, this height difference was 6 cm, corresponding to a typical height difference for a female user during catheterisation. Bladder tissue is illustrated by reference number 231.

Figure 27 illustrates the flow-rate through a catheter as a function of the total area of the drainage openings. Curves for three different catheter sizes (CH10, CH12 and CH16) are shown in the figure. The top curve is the curve for a CH16 catheter, the middle curve is the curve for a CH12 catheter and the lowest curve is the curve for a CH10 catheter. The figure illustrates that the flow-rate converges approximately when the total area of the drainage openings (the total inflow area) are of the same size as the cross-sectional area of the inner lumen of the catheter. This is shown by the vertical lines drawn from where the curves flattens and to the X-axis. The cross-sectional areal of the inner lumen of a CH16 is approximately 11 mm$^2$, of a CH12 approximately 5.5 mm$^2$ and for a CH10, approximately 4 mm$^2$. See also Example 3 above.

Figures 28 to 29 illustrate comparison between a prototype catheter having 144 drainage openings positioned as shown in figure 14 and a prior art catheter having two larger drainage openings. Figure 28 illustrates the number of clogging events after 10 tests with each catheter. The left bar and the right bar are results from testing on a prior art catheter and the four in the middle are results from testing on a prototype catheter. Figure 29 illustrates the average amount of residual urine left in the bladder after 10 tests. The bladder was filled with 200 ml of water, so 40 g (the left bar) corresponds to 20%. Again, the left bar and the right bar are results from testing on a prior art catheter and the four in the middle are results from testing on a prototype catheter. The results show that a prototype catheter having 144 drainage openings never clogs during the test (see figure 28) and leaves no residual urine in the bladder (see figure 29). In comparison, a prior art catheter shows many clogging events (figure 28) and also that residual urine is left in the bladder after a first flow stop.

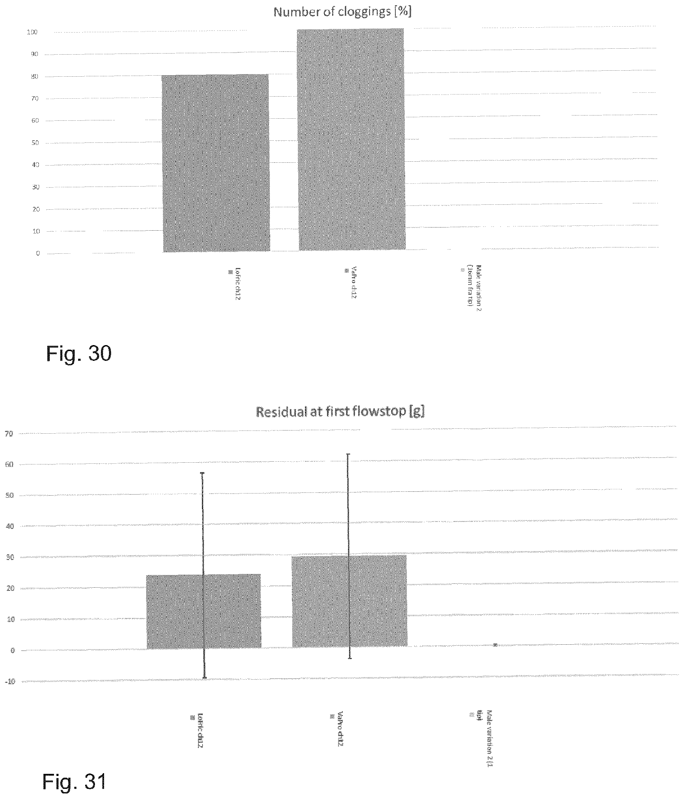

Figure 30 and 31 illustrates a comparison between two prior art catheters (LoFric ® available from Dentsply IH and VaPro ® available from Hollister Inc.) and a prototype catheter having 144 drainage openings. Figure 30 illustrates the number of cloggings occurring during 10 tests and figure 31 illustrates the average amount of residual urine left after a first flow stop. In figure 30 the first two bars relate to tests on LoFric® and VaPro® respectively, the third bar (which is "0") represents the result of testing the prototype catheter. It is clear from figure 30 that the prototype catheter never clogs - the flow is never completely stopped during draining of the bladder. In figure 31, the bars have the same meaning, i.e. the two first relates to testing of LoFric and VaPro respectively and the third bar (again "0") is the result of testing of the prototype catheter. From figure 31 it is clear that the amount of residual urine is nil (nothing) when using a prototype catheter, whereas the prior art catheters always leaves residual urine at a first flow stop.

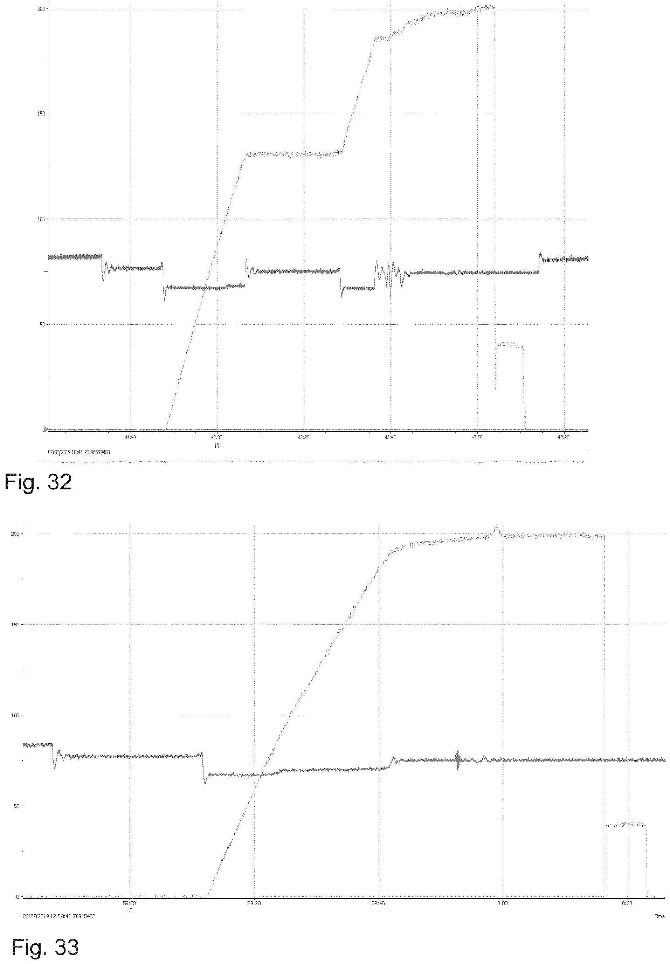

Figure 32 to 36 illustrate flow/pressure graphs from testing of prior art catheters and prototype catheters in a porcine-bladder as described below. Figure 32 illustrate testing of a prior art catheter. The curve starting at the bottom left and increasing toward the right corner is the flow through the catheter and the curve at the middle is the pressure. The bladder was filled with approximately 200 ml of water, so the flow starts at zero and ends at approximately 200 ml. A flow stop is visible on the flow-curve as the horizontal part after draining approximately 130 ml of liquid. The pressure-curve indicates fluctuations at this point - this is because the catheter had to be re-positioned so that the draining could continue. Further flow-stops occurred toward the end of the emptying procedure, from approximately 180 ml emptying and onwards. This is visible as horizontal steps on the flow curve and as fluctuations due to repositioning at the pressure-curve.

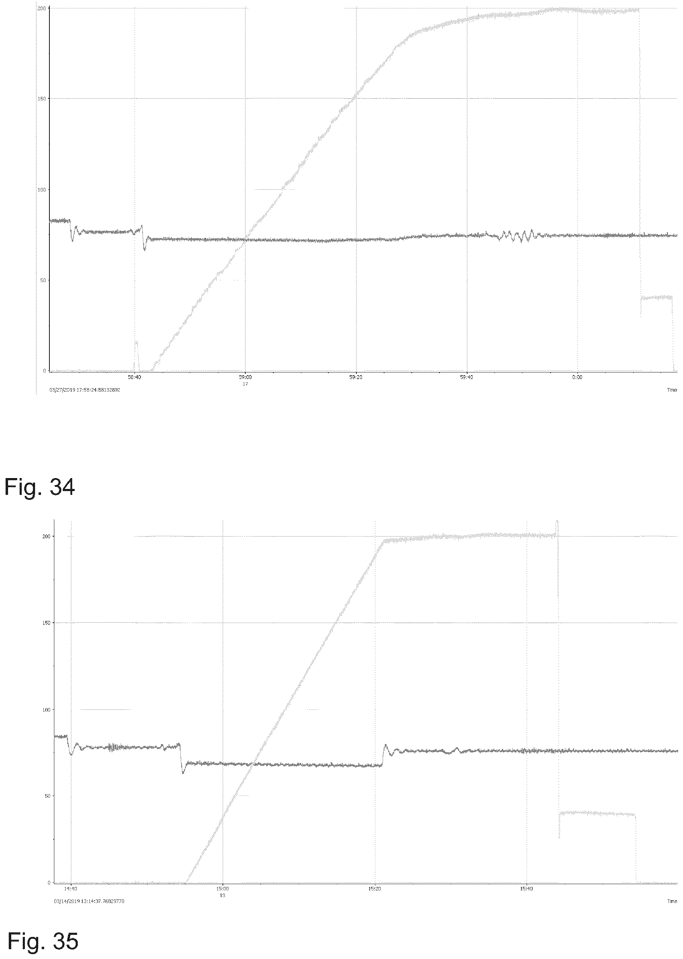

Figure 33 to 35 illustrate testing of three of the 36 prototypes mentioned below. Only three are shown but testing of all of the 36 prototypes led to similar results. Figure 33 illustrate testing of prototype number 9, figure 34 illustrates testing of prototype number 24 and figure 35 illustrates testing of prototype number 29. Prototype 9 had 24 drainage openings each having a diameter of 0.8 mm. The drainage openings were positioned in two rows with 180 degrees between them around the circumference of the catheter with 2.0 mm between them in longitudinal direction. This means that the drainage openings were positioned in two oppositely facing rows with 12 drainage openings in each row. Prototype 24 had 48 drainage openings each having a diameter of 0.4 mm. The drainage openings were positioned in four rows with 90 degrees between them around the circumference of the catheter with 2.0 mm between them in longitudinal direction. This means 12 drainage openings in the longitudinal direction and four around the circumference with 90 degrees between them. Prototype 29 had 12 drainage openings each having a diameter of 0.4 mm. The drainage openings were positioned in three rows with 120 degrees between them around the circumference of the catheter and with 2.0 mm between them in longitudinal direction. This means 4 drainage openings in the longitudinal direction and three around the circumference with 120 degrees between them. From these three examples it is clear that the flow using a prototype catheter is continuous until the bladder is completely drained. There are no horizontal parts on the flow-curves. Therefore, there is no need for repositioning of the catheter and thus no fluctuations in the pressure-curve during the emptying. Fluctuations occurs at the very end, when the bladder is empty - these illustrate that the catheter is being removed from the bladder.

Figure 36 illustrate flow-/pressure-curves for testing of LoFric® and VaPro® compared with a prototype catheter having 144 drainage openings. These flow-/pressure-curves correspond to the results shown in figures 30 and 31. The first curve to the left is the result of testing on the LoFric® catheter, the second curve is the result of testing on VaPro® and the third curve is the result of testing on the prototype catheter. It is clear that both prior art catheters show a flow-stop during testing - there is a part on the flow-curve that is horizontal. It is also clear that this does not happen with the prototype catheter - there is no horizontal part on the flow-curve for that catheter.

Figures 37A and 37B illustrate schematically the inflow into a catheter having only two large drainage openings (figure 37A) and into a catheter having multiple smaller drainage openings (figure 37B). In figure 37A - the prior art catheter - the inflow into the catheter occurs mainly through the drainage opening positioned lowest in the bladder during draining - typically the most distal drainage opening. This is illustrated by the larger arrow. In figure 37B, the inflow into the drainage openings are more evenly distributed and even thought the inflow into the lower drainage openings are larger than the inflow into the drainage openings positioned higher in the bladder, the difference is much less pronounced.

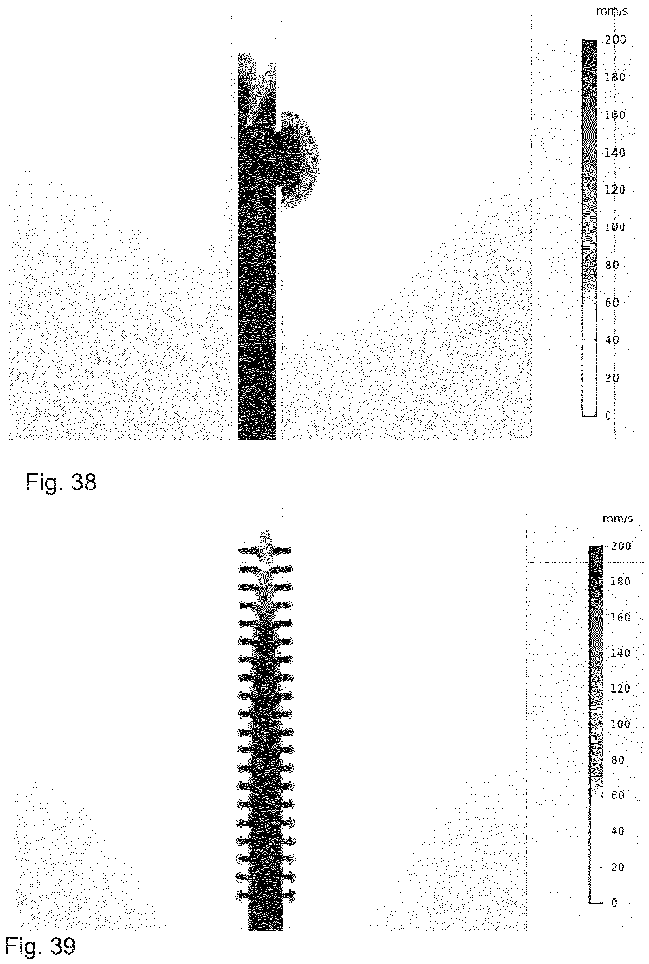

Figures 38 and 39 illustrate simulations of flow of urine into an intermittent catheter in a bladder during draining. Figure 38 illustrates the flow-profile of an intermittent catheter having only one large drainage opening - i.e. a catheter not forming part of this invention. Figure 39 illustrates the flow-profile of an intermittent catheter having 20 small drainage openings and where the total inflow area does not exceed the cross-sectional area of the lumen of the catheter distally of the most distal drainage opening. That the total inflow area does not exceed the cross-sectional area of the lumen of the catheter means that there is inflow into all of the drainage openings - which is also evident in the figure. The figures illustrate how far from the catheter the flow in the bladder is influenced from the suction force provided by the catheter. Figure 38 illustrates that the flow in the bladder is influenced in a distance corresponding roughly to the diameter of the catheter from the drainage opening. In figure 39, the flow is influenced in a distance corresponding to approximately $1/10^{th}$ of the diameter of the catheter.

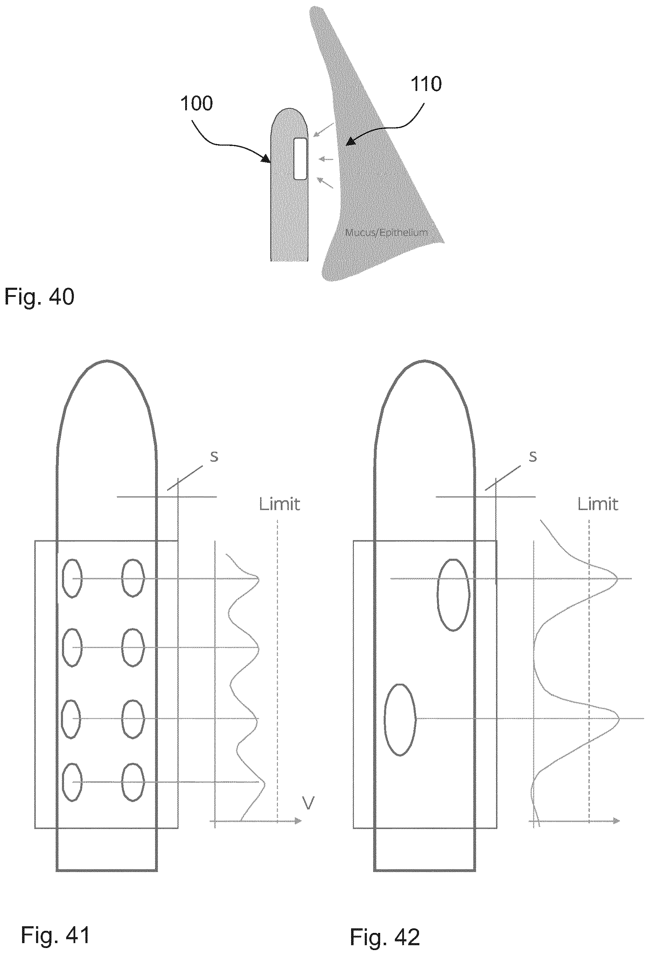

Figure 40 illustrates schematically, how the bladder wall **110** may be drawn towards a catheter **100** during a catheterisation procedure. This is the result of the pressure drop in the bladder due to the draining of the urine.

Figure 41 and 42 illustrate schematically the flow-rate around the drainage openings. In the figures, the flow rate is illustrated as a curve to the right of the figures, which has an increase at the drainage openings. It is clear that the flow rate is smaller, when there are more drainage openings, as in figure 41, than when only two drainage larger drainage openings is present as in figure 42.

Figures 43 to 61 Illustrate simulations of flow according to different configurations of drainage openings and objects close to the catheter during draining.

Figure 43 illustrates simulated flow through a prior art catheter 400 having eyelets 401, 402, of a size of 2.5 mm² positioned 20 mm apart from each other. The catheter is simulated as being a CH12 catheter. The darker colours 403 in the figure illustrates higher flow-rate. The figure illustrates that the flow-rate is higher from the lower eyelet 402 than from the higher eyelet 401 - which is also what is expected. Furthermore, the inflow volume in the bladder is indicated by the lighter coloured area 404 in the vicinity of the eyelets.

Figures 44 and 45 illustrate simulated flow through a prior art catheter 400 having one eyelet 401 of 2.5 mm². This simulates the situation, where one of eyelets in a prior art catheter with two eyelets is blocked, e.g. due to bladder tissue having entered into the eyelet during the voiding procedure. In the simulated procedure, a wall 405 is positioned 5 mm from the catheter 400. Figure 44 illustrates a side-view of the simulation, whereas figure 45 illustrates a perspective view. In figure 44, a darker area 406 is illustrated on the wall 405. This is a simulation of the impact in form of drag-force on the wall resulting from the flow-rate through the eyelet 401.

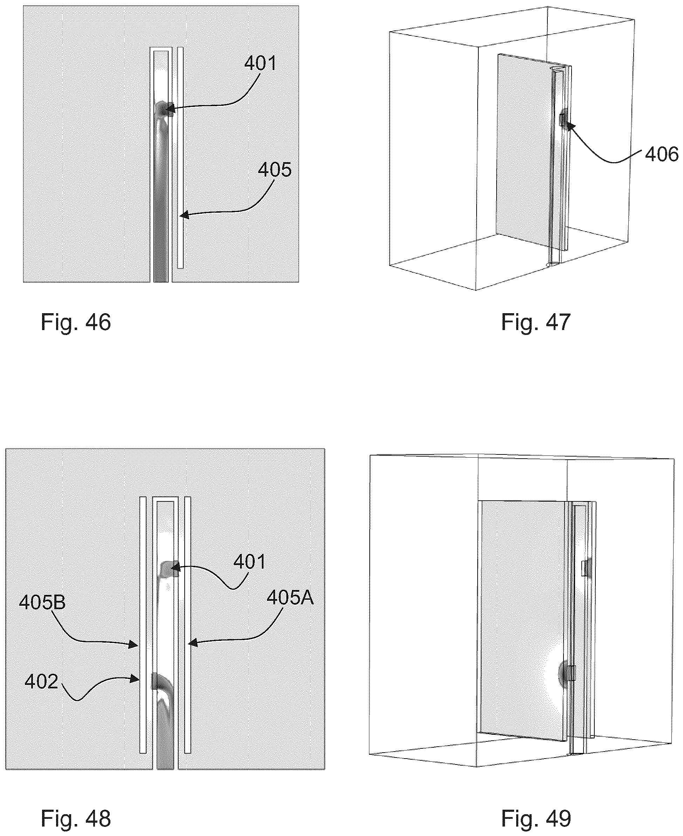

Figures 46 and 47 illustrate similar simulations as figures 45 and 46. The only difference is that in this simulation, the wall 405 is positioned 1mm from the catheter. In figure 46, it is clear that the darker coloured area 406 illustrating the impacted area is smaller. However, the force per area-unit is higher, which is also visible as the colour is darker in the centre of the area.

Figures 48 and 49 illustrate a simulation, where two walls 405A, 405B, are positioned 1 mm from the catheter - one on each side.

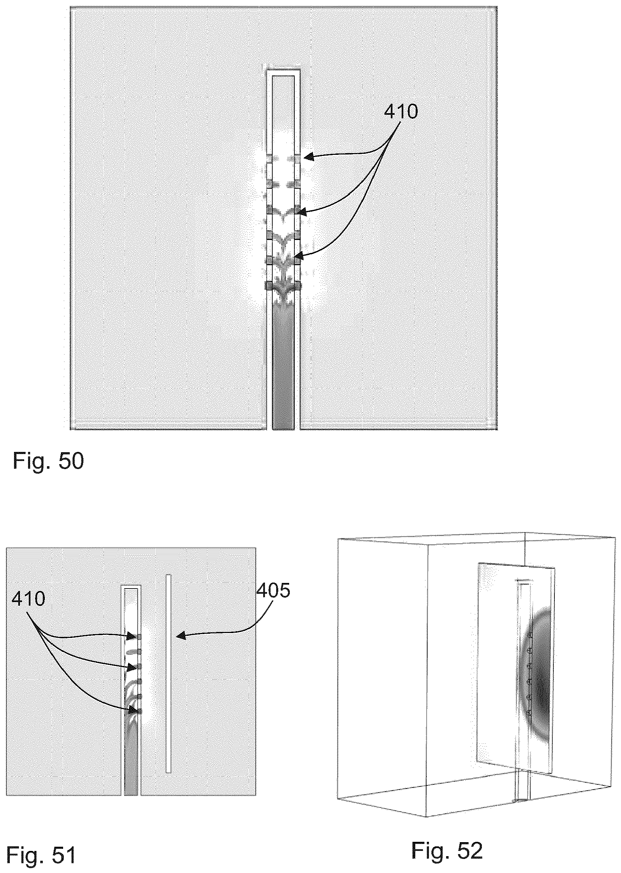

Figure 50 illustrates simulation of a flow-profile in an intermittent catheter according to embodiments of this disclosure. The catheter in figure 50 has 12 drainage openings 410, positioned 6 on each side. Each drainage opening is 1 mm in diameter and they are positioned 3mm apart in longitudinal direction. The flow-profile illustrates inflow into each drainage opening and that the inflow is higher in the lower positioned drainage openings.

Figures 51 and 52 illustrate simulation of flow in a situation where the drainage openings 410 on only one side are open and a wall 405 is positioned 5 mm from the catheter facing the open drainage openings. Figure 51 illustrates a side-view and figure 52 illustrates a perspective view of the simulation. In figure 51, it is apparent that a rather large area of the wall is impacted by the inflow - but the colours are not very dark, hence the impact is small.

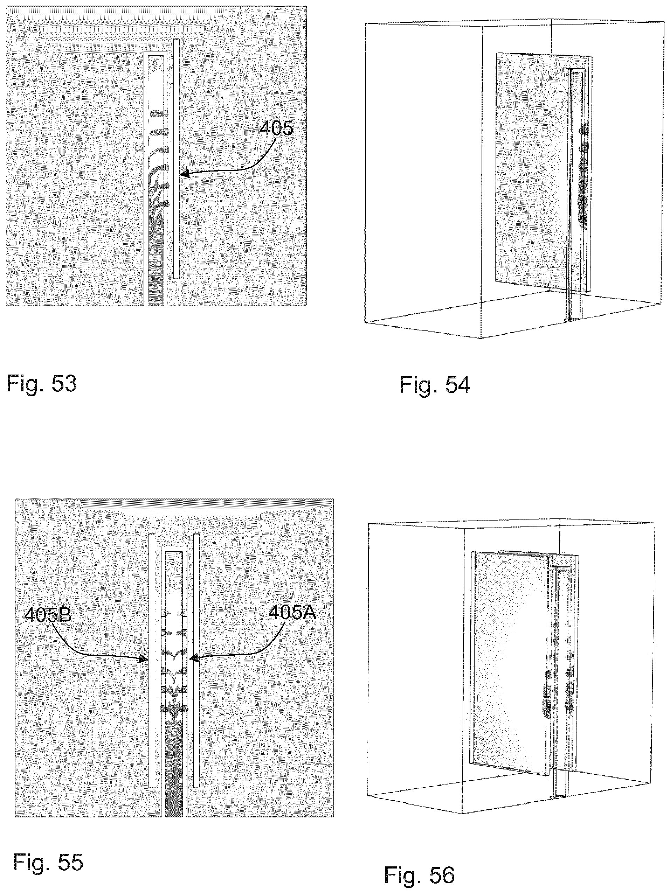

Figures 53 and 54 illustrate similar simulations of flow. The only difference is that in this simulation, the wall 405 is positioned 1 mm from the catheter facing the opening drainage openings 410.

Figures 55 and 56 illustrate simulations of flow in a situation where two walls, 405A, 405B are positioned one on each side of the catheter.

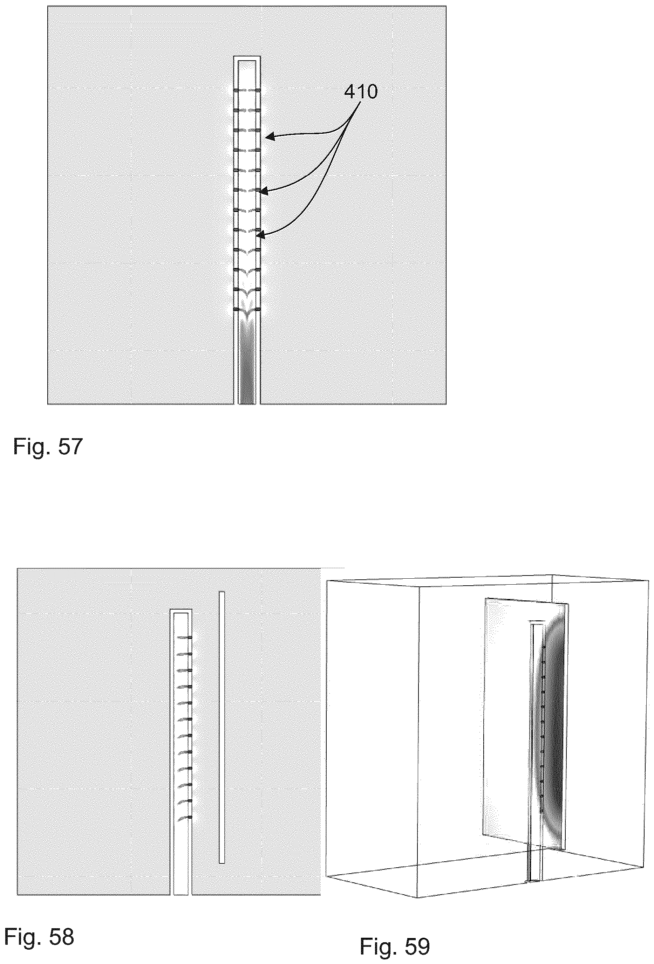

Figure 57 illustrates simulates of a flow-profile in an intermittent catheter according to embodiments of this disclosure. The catheter in figure 57 has 24 drainage openings 410 positioned 12 on each side. Each drainage opening is 0.4 mm in diameter and they are positioned 3 mm apart in longitudinal direction. The flow-profile illustrates inflow into each drainage opening.

Figures 58 and 59 illustrate simulation of flow in a situation where the drainage openings 410 on only one side are open and a wall 405 is positioned 5 mm from the catheter facing the open drainage openings. Figure 58 illustrates a side-view and figure 59 illustrates a perspective view of the simulation. In figure 58, it is apparent that a rather large area of the wall is impacted by the inflow - but the colours are not very dark, hence the impact is small.

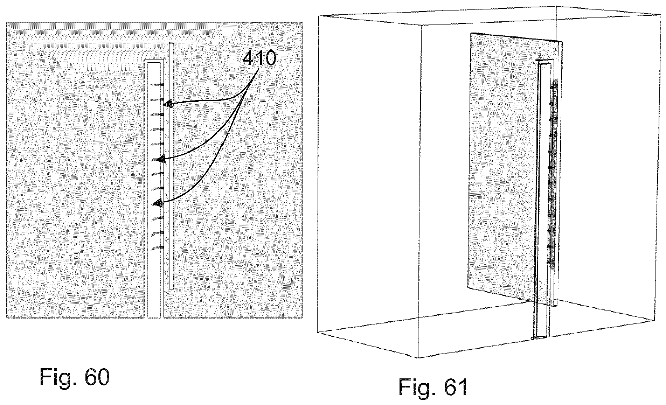

Figures 60 and 61 illustrate similar simulations of flow. The only difference is that in this simulation, the wall 405 is positioned 1 mm from the catheter facing the opening drainage openings 410.

Figures 62 and 63 illustrate test set-ups that can be used for determining the drag-force (figure 62) and suction pressure profiles (figure 63).

A container **1001** is provided to simulate a full bladder. The container **1001** is filled with water **1002**. On top of the container **1001**, a scanner motor **1003** is provided. The scanner motor **1003** is connected to a holding device **1004.** A catheter **1005** is inserted through the bottom of the container. In the illustrated test set-up the catheter **1005** is a prior art catheter being provided with two large drainage openings **1006.** The catheter is inserted into the container **1001** through a liquid-tight connection **1007.** In order to prolong the period of measuring, the container **1001** may be equipped with a pump **1008,** that recycles what is drained through the catheter.

In figure 62, the measuring device is illustrated as being a load cell **1009** connected to a plate **1010** simulating an object (e.g. a bladder wall) close to the drainage opening.

In figure 63, the measuring device is illustrated as being pressure sensor **1011.**

Figure 64 illustrates a schematic view of the suction pressure profile on a prior art catheter having only two large drainage openings. The catheter is as described in configuration 1 under Example 6 above. The figure illustrates the pressure determined 1 mm externally of the catheter transversely out from the drainage opening.

Figure 65 illustrates simulation of suction pressure 1 mm externally of the drainage opening in the same catheter as in figure 64. In this figure, the suction pressure in an axial direction of the drainage opening is illustrated, i.e. zero degrees from the drainage opening, corresponding to case 6 in Example 6 above. Figure 65 illustrates simulation of suction pressure at the upper drainage opening under two different flow-rates, 2 ml/s and 10 ml/s. Under a flow-rate of 10 ml/s, the maximum pressure exceeds 45 Pa. Only the drainage portion defined as being from the most distal drainage opening to the most proximal drainage opening is considered. Staying within this drainage portion, it is further clear that the minimal pressure occurs in a distance away from the drainage opening - in this two-dimensional view downwards. The minimal pressure is seen as being zero. Therefore, a pressure ratio as defined above will in this case be infinite.

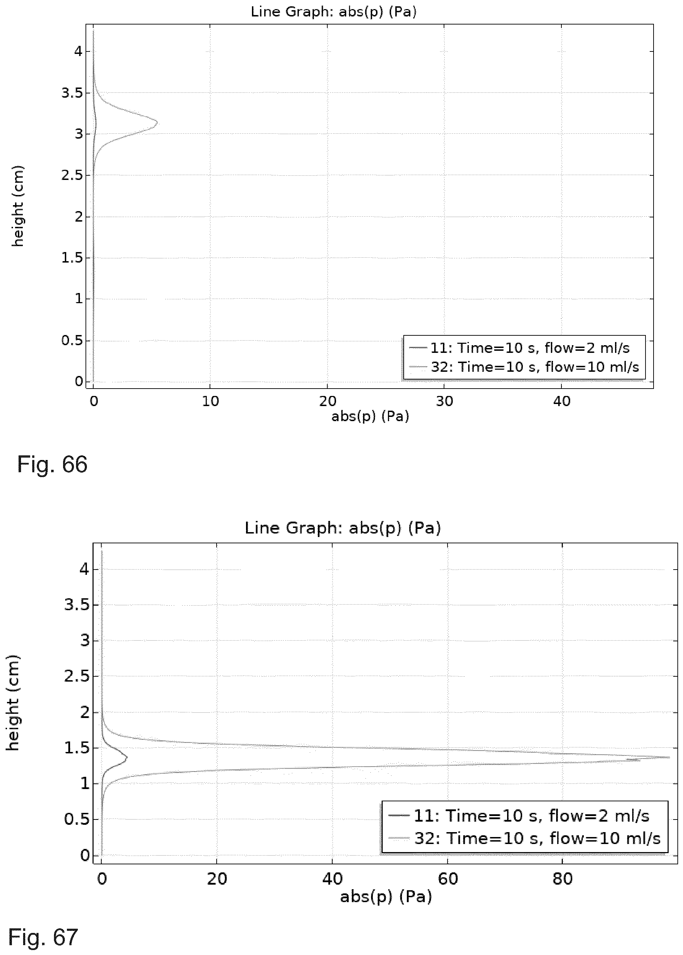

Figure 66 also illustrates simulation of suction pressure 1 mm externally of the drainage opening in the same catheter as in figure 64. In this figure, the suction 45 degrees around the circumference is illustrated, corresponding to case 7 in Example 6 above. Like with figure 65, figure 66 illustrates simulation of suction pressure at the upper drainage opening under two different flow-rates, 2 ml/s and 10 ml/s. Under a flow-rate of 10 ml/s, the maximum pressure is around 5 Pa. It is further clear that the minimal pressure occurs in a distance away from the drainage opening - downwards in this two-dimensional view. The minimal pressure is seen as being zero. As before, with figure 65, a pressure ration will in this case be infinite.

Figure 67 illustrates suction pressure 1 mm externally of the drainage opening in the same catheter as in figure 64. Like with figure 65, in this figure, the suction pressure in an axial direction of the drainage opening is illustrated, i.e. zero degrees from the drainage opening, corresponding to case 6 in Example 6 above. Figure 65 illustrates simulation of suction pressure at the lower drainage opening under two different flow-rates, 2 ml/s and 10 ml/s. Under a flow-rate of 10 ml/s, the maximum pressure gets close to 100 P$\alpha$. It is further clear that the minimal pressure occurs in a distance downwards from the drainage opening. The minimal pressure is seen as being zero. Therefore, a pressure ratio as defined above will also in this case be infinite.

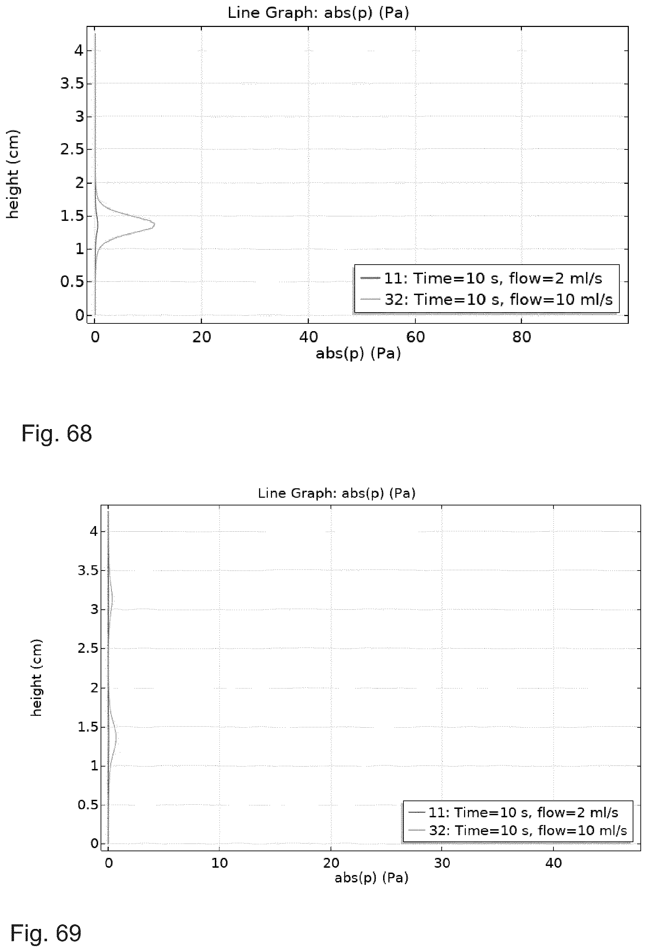

Figure 68 is similar to figure 66, the suction pressure 1 mm externally of the catheter and 45 degrees around the circumference with respect to the drainage opening, is simulated. The maximum pressure is around 12 P$\alpha$. Like before, the minimal pressure occurs in a distance away from the drainage opening, and is seen as being zero, meaning that also in this case, the pressure ratio as defined above will be infinite.

Figure 69 illustrates simulation of suction pressure on the same catheter as in figure 64, and in a distance of 1mm externally of the catheter. In this simulation, the suction pressure 90 degrees around the circumference from the drainage openings is determined. This corresponds to case 8 above in Example 6. Both the upper and lower drainage opening contribute in this simulation - however, the suction pressure at the longitudinal position of the drainage openings are very small compared to the other simulations. The suction pressure is in both cases close to or less than 1 Pa. However, since the suction pressure between the drainage openings is zero, the pressure ratio as defined above will still be infinite.

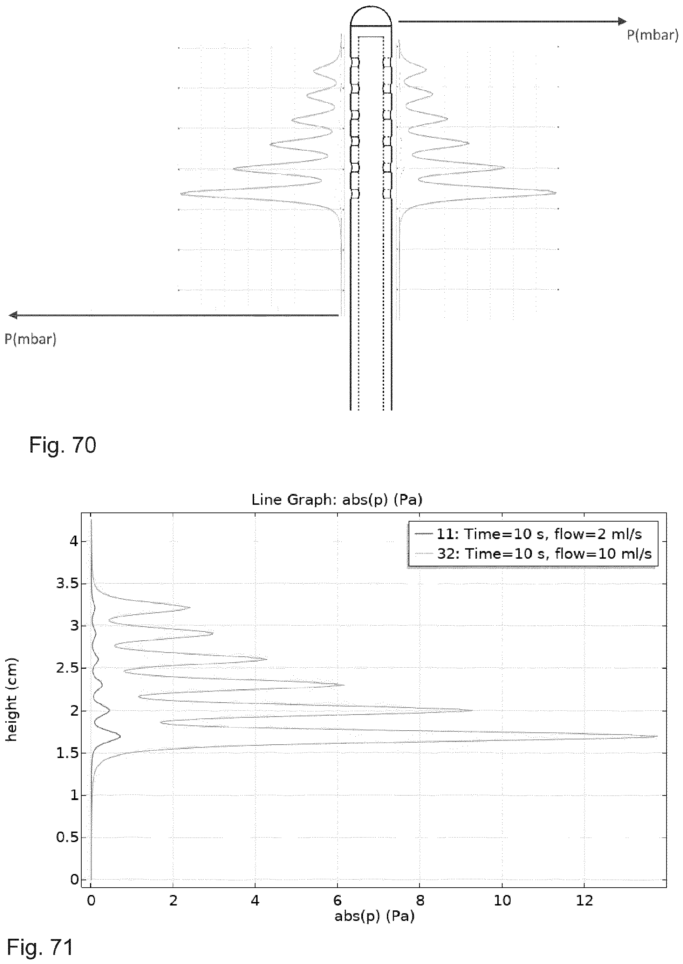

Figure 70 illustrates a schematic view of the suction pressure profile on an intermittent urinary catheter having 12 drainage openings, each having a diameter of 1 mm. The catheter is as described in configuration 2 under Example 6 above. The figure illustrates the pressure determined 1 mm externally of the catheter transversely out from the drainage opening.

Figure 71 illustrates simulation of suction pressure 1 mm externally of the drainage opening in the same catheter as in figure 70. In this figure, the suction pressure in an axial direction of the drainage opening is illustrated, i.e. zero degrees from the drainage opening, corresponding to case 6 in Example 6 above. Figure 71 illustrates simulation of suction pressure at the drainage openings under two different flow-rates, 2 ml/s and 10 ml/s. Under a flow-rate of 10 ml/s, the maximum pressure reaches around 14 P$\alpha$. We will only consider the drainage portion of the catheter in these simulations, meaning from the most proximal drainage opening to the most distal drainage opening. Within this drainage portion, it is further clear that the minimal pressure occurs in between the drainage openings; the lowest value seems to be around 0.5 Pa. Therefore, a pressure ratio as defined above will in this case be 28 along this row of drainage openings. This is considered to be acceptable for a longitudinal row of drainage openings.

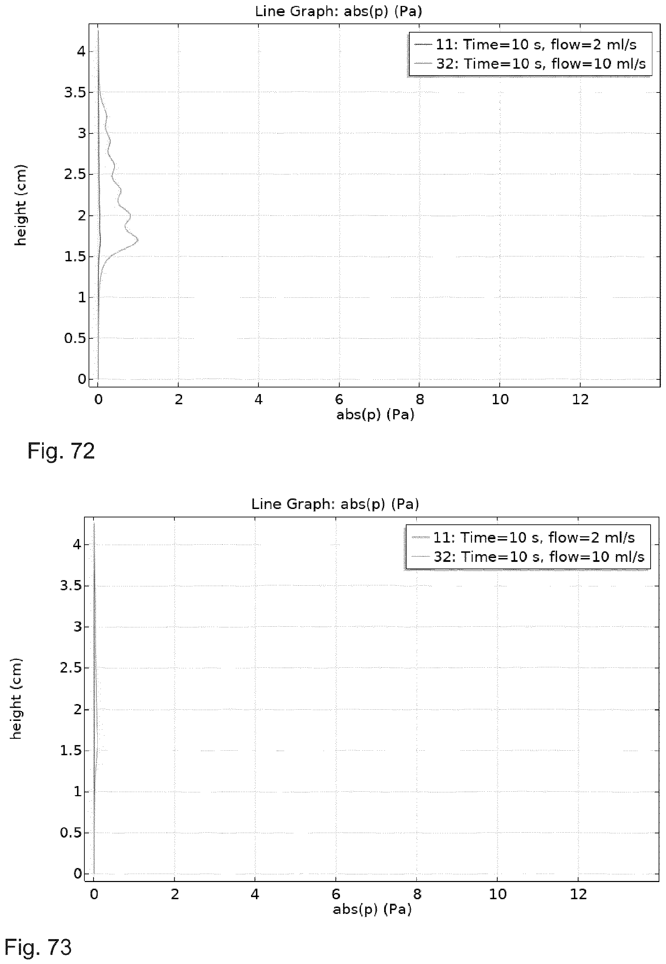

Figure 72 also illustrates simulation of suction pressure 1 mm externally of the drainage opening in the same catheter as in figure 70. In this figure, the suction 45 degrees around the circumference is illustrated, corresponding to case 7 in Example 6 above. Again simulation of suction pressure at the drainage openings under two different flow-rates, 2 ml/s and 10 ml/s is illustrated. Under a flow-rate of 10 ml/s, the maximum pressure is around 1 Pa. It is further clear that the minimal pressure occurs towards the upper drainage opening and seems to be around 0.2 Pa. If the minimal suction pressure of 0.2 Pa is compared to the maximum suction pressure in a direction of the drainage openings, which was 14 Pa (see figure 71), then the pressure ratio will be around 70, which is considered to be undesirable.

Figure 73 illustrates simulation of suction pressure on the same catheter as in figure 70, and in a distance of 1mm externally of the catheter. In this simulation, the suction pressure 90 degrees around the circumference from the drainage openings is determined. This corresponds to case 8 above in Example 6. The suction pressure at the longitudinal position of the drainage openings are very small compared to the other simulations in figure 71 and 72. The suction pressure seems to be less than 0.1 Pa. Again comparing this value to the maximum suction pressure of 14 Pa (figure 71), then the pressure ratio will be around 140, which is considered to be undesirable.

It may seem that an intermittent urinary catheter having 12 drainage openings of a size of 1 mm in diameter provides a suction pressure profile, which is undesirable. However, this is not necessarily the case. In the above simulations, only two longitudinal rows were used. It is contemplated that providing more longitudinal rows, will reduce the difference between the maximum suction pressure and the minimal suction pressure within the drainage portion - thus, reduce the pressure ratio to an acceptable level.

Figure 74 illustrates a schematic view of the suction pressure profile on an intermittent urinary catheter having 24 drainage openings, each having a diameter of 0.4 mm. The catheter is as described in configuration 3 under Example 6 above. The figure illustrates the pressure determined 1 mm externally of the catheter transversely out from the drainage opening.

Figure 75 illustrates simulation of suction pressure 1 mm externally of the drainage opening in the same catheter as in figure 74. In this figure, the suction pressure in an axial direction of the drainage opening is illustrated, i.e. zero degrees from the drainage opening, corresponding to case 6 in Example 6 above. Figure 74 illustrates simulation of suction pressure at the drainage openings under two different flow-rates, 1 ml/s and 5 ml/s, thereby making the simulation to be similar to one run on a catheter having 48 drainage openings under flow rates of 2 ml/s and 10ml/s respectively. However, for illustrative purposes - to be able to distinguish the drainage openings from each other, this simulation using 24 drainage openings and half the flow rate was used instead. Under a flow-rate of 5 ml/s, the maximum pressure reaches around 0.7 Pa. We will only consider the drainage portion of the catheter in these simulations, meaning from the most proximal drainage opening to the most distal drainage opening. Within this drainage portion, it is further clear that the minimal pressure occurs in between the drainage openings; the lowest value seems to be around 0.05 Pa. Therefore, a pressure ratio as defined above will in this case be 14 along this row of drainage openings. This is considered to be acceptable for a longitudinal row of drainage openings.

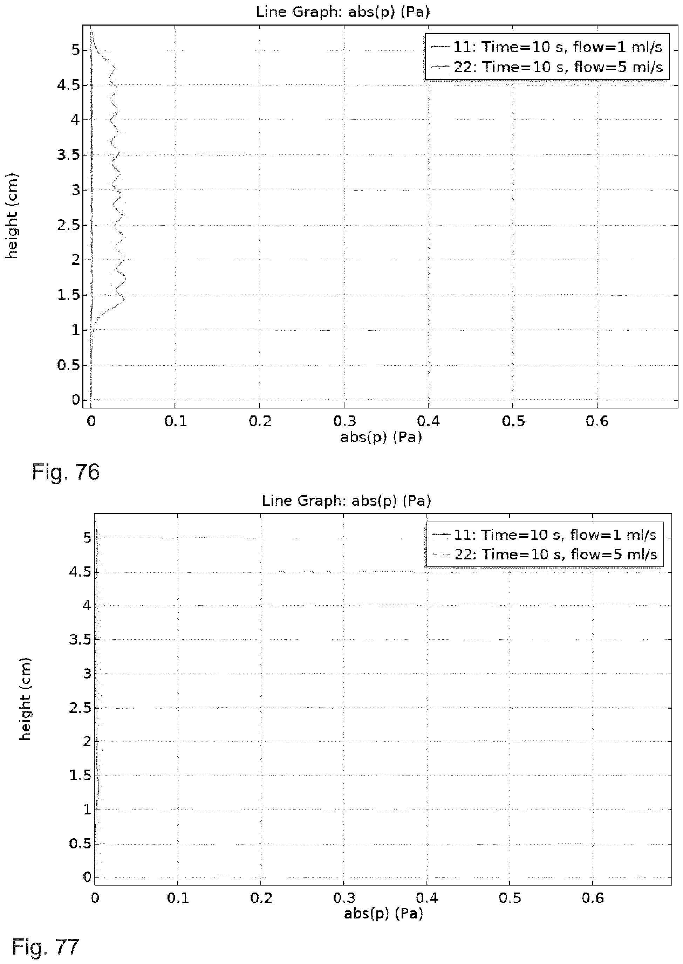

Figure 76 also illustrates simulation of suction pressure 1 mm externally of the drainage opening in the same catheter as in figure 74. In this figure, the suction 45 degrees around the circumference is illustrated, corresponding to case 7 in Example 6 above. Again simulation of suction pressure at the drainage openings under two different flow-rates, 1 ml/s and 5 ml/s is illustrated. Under a flow-rate of 5 ml/s, the maximum pressure is around 0.05 P$\alpha$. It is further clear that the suction pressure is very evenly distributed. The pressure ratio will be around 14, compared to the maximum suction pressure occurring in a direction of the drainage openings. This is considered to be acceptable.

Figure 77 illustrates simulation of suction pressure on the same catheter as in figure 74, and in a distance of 1mm externally of the catheter. In this simulation, the suction pressure 90 degrees around the circumference from the drainage openings is determined. This corresponds to case 8 above in Example 6. The suction pressure at the longitudinal position of the drainage openings are close to 0.01 Pa. Again comparing this value to the maximum suction pressure of 0.7 Pa (figure 75), then the pressure ratio will be around 70, which is considered within the desired limit.

It may seem that even an intermittent urinary catheter having 48 drainage openings of a size of 0.4 mm in diameter provides a suction pressure profile, which is undesirable. However, this is not necessarily the case. In the above simulations, only two longitudinal rows were used. It is contemplated that providing more longitudinal rows, will reduce the difference between the maximum suction pressure and the minimal suction pressure within the drainage portion - thus, reduce the pressure ratio to an acceptable level. Particularly, since it seems that even with only two longitudinal rows, an acceptable suction pressure profile is achieved.

Figures 62 and 63 illustrate test set-ups that can be used for determining the drag-force (figure 62) and suction pressure profiles (figure 63).

A container **1001** is provided to simulate a full bladder. The container **1001** is filled with water **1002**. On top of the container **1001,** a scanner motor **1003** is provided. The scanner motor **1003** is connected to a holding device **1004.** A catheter **1005** is inserted through the bottom of the container. In the illustrated test set-up the catheter **1005** is a prior art catheter being provided with two large drainage openings **1006**. The catheter is inserted into the container **1001** through a liquid-tight connection **1007.** In order to prolong the period of measuring, the container **1001** may be equipped with a pump **1008,** that recycles what is drained through the catheter.

In figure 62, the measuring device is illustrated as being a load cell **1009** connected to a plate **1010** simulating an object (e.g. a bladder wall) close to the drainage opening.

In figure 63, the measuring device is illustrated as being pressure sensor **1011.**

**Embodiments**

1. An intermittent urinary catheter having a tip portion with a tip in a proximal insertion end of the catheter, a tubular portion extending from the tip portion to a distal outlet end; the tubular portion having an inside lumen and a drainage portion provided with drainage openings configured for allowing urine to enter the inside lumen, the drainage portion being provided with more than 12 drainage openings.

2. An intermittent urinary catheter having a tip portion with a tip in a proximal insertion end of the catheter, a tubular portion extending from the tip portion to a distal outlet end; the tubular portion having an inside lumen and a drainage portion provided with drainage openings configured for allowing urine to enter the inside lumen, the drainage portion being provided with drainage openings each having a cross-sectional area of less than 0.4 mm$^2$.

3. An intermittent urinary catheter having a tip portion with a tip in a proximal insertion end of the catheter, a tubular portion extending from the tip portion to a distal outlet end, the tubular portion having an inside lumen and drainage portion provided with drainage openings configured for allowing urine to enter the inside lumen, a number and size the of drainage openings being configured for providing a non-intermittent flow.

4. An intermittent urinary catheter having a tip portion with a tip in a proximal insertion end of the catheter, a tubular portion extending from the tip portion to a distal outlet end, the tubular portion having an inside lumen and drainage portion provided with drainage openings configured for allowing urine to enter the inside lumen, the catheter being configured such that the pressure pulse in the lumen does not exceed a predetermined threshold value when tested as described herein.

5. An intermittent urinary catheter having a tip portion with a tip in a proximal insertion end of the catheter, a tubular portion extending from the tip portion to a distal outlet end, the tubular portion having an inside lumen and drainage portion provided with drainage openings configured for allowing urine to enter the inside lumen, the drainage openings being configured to reduce the pressure ratio between a maximum suction pressure and a minimal suction pressure, where the maximum and minimal suction pressure are determined in a distance of 1 mm externally of the drainage portion of the catheter.

6. An intermittent urinary catheter having a tip portion with a tip in a proximal insertion end of the catheter, a tubular portion extending from the tip portion to a distal outlet end, the tubular portion having an inside lumen and drainage portion provided with drainage openings configured for allowing urine to enter the inside lumen, the drainage openings being positioned in longitudinal rows and being configured to reduce the pressure ratio between maximum suction pressure and minimal suction pressure, where the maximum and minimal suction pressure are determined in a distance of 1 mm externally of the longitudinal rows.

7. An intermittent urinary catheter having a tip portion with a tip in a proximal insertion end of the catheter, a tubular portion extending from the tip portion to a distal outlet end, the tubular portion having an inside lumen and drainage portion provided with drainage openings configured for allowing urine to enter the inside lumen, a number and size the of drainage openings being configured for providing an even distribution of inflow through the drainage openings, where the even distribution of inflow is defined as being within a factor 70 between a maximum flow-rate transversely with respect to a drainage opening and a minimal flow-rate at any point between the drainage openings.

8. An intermittent urinary catheter having a tip portion with a tip in a proximal insertion end of the catheter, a tubular portion extending from the tip portion to a distal outlet end, the tubular portion having an inside lumen and drainage portion provided with drainage openings configured for allowing urine to enter the inside lumen, the drainage openings being configured for providing a drag-force of less than 1 mN in a distance of 1 mm from the drainage opening, when a flow rate through the catheter is in the range of 2 ml/s to 10 ml/s.

9. An intermittent urinary catheter having a tip portion with a tip in a proximal insertion end of the catheter, a tubular portion extending from the tip portion to a distal outlet end, the tubular portion having an inside lumen and drainage portion provided with drainage openings configured for allowing urine to enter the inside lumen, the drainage portion having a degree of perforation of between 0.01 and 0.6.

10. A catheter comprising:

an intermittent urinary catheter having a tubular portion extending from a proximal insertion end to a distal outlet end, with the tubular portion formed to include a lumen adapted to transport urine through the intermittent urinary catheter;

a drainage area provided on an exterior surface of the tubular portion, where the drainage area includes a plurality of drainage openings that combine to provide an open drain area;

wherein the drainage area is defined by a length measured form a proximal edge of a proximal most one of the plurality of the drainage openings that is closest to the proximal insertion end to a distal edge of a distal most one of the plurality of the drainage openings that is closest to the distal outlet end multiplied by a circumference of the tubular portion measured within the length of the drainage area;

wherein a ratio of the open drain area to the drainage area on the exterior surface of the tubular portion is in a range from 0.05 to 0.7.

11. A catheter comprising:

an intermittent urinary catheter having a tubular portion extending from a proximal insertion end to a distal outlet end, with the tubular portion formed to include a lumen adapted to transport urine through the intermittent urinary catheter;

a drainage area provided on an exterior surface of the tubular portion, where the drainage area includes a closed surface area and an open surface area, where the open surface area of the drainage area allows urine to enter the lumen;

wherein the drainage area is defined by a length measured as a longitudinal distance from a proximal most edge of the open surface area to a distal most edge of the open surface area multiplied by a circumference of the tubular portion measured within the length of the drainage area;

wherein the open surface area comprises from 5% to 70% of the drainage area on the exterior surface of the tubular portion.

12. An intermittent urinary catheter having a tip portion with a tip in a proximal insertion end of the catheter, a tubular portion extending from the tip portion to a distal outlet end; the tubular portion having an inside lumen and a drainage portion provided with drainage openings configured for allowing urine to enter the inside lumen, wherein the largest dimension of any of the drainage openings is less than 1mm and the total inflow area of the drainage openings is larger than the cross-sectional area of the inside lumen of the catheter.

13. The intermittent urinary catheter as in embodiment 4, wherein the threshold limit is 50mBar when tested as described herein in Example 1, under 10 cm water column with a height difference of 10-15 cm between the drainage opening and a catheter outlet.

14. The intermittent urinary catheter as in embodiment 4, wherein the threshold limit is 350mBar when tested as described herein in Example 2, under 50 cm water column with a height difference of 25cm between the drainage opening and a catheter outlet.

15. The intermittent urinary catheter as in embodiment 4, wherein the threshold limit is 300mBar when tested as described herein in Example 2, under 50 cm water column with a height difference of 6 cm between the drainage opening and a catheter outlet.

16. The intermittent urinary catheter as in any of the preceding embodiments, wherein the tubular portion defines a convex outer surface, and wherein the total inflow area of the drainage openings in the convex outside surface of the tubular portion is larger than a cross-sectional area of the inside lumen of the catheter in a cross section perpendicular to a longitudinal direction of the tubular portion at a position distally of the drainage openings.

17. The intermittent urinary catheter as in any of the preceding embodiments, wherein the tubular portion defines a convex outer surface, and wherein the total inflow area of the drainage openings in the convex outside surface of the tubular portion is larger than twice the cross-sectional area of the inside lumen of the catheter in a cross section perpendicular to a longitudinal direction of the tubular portion at a position distally of the drainage openings.

18. The intermittent urinary catheter as in any of the preceding embodiments, wherein the number of drainage openings is higher than required for filling the lumen just distally of the drainage openings.

19. The intermittent urinary catheter as in any of the preceding embodiments, wherein the number of drainage openings is higher than a first predetermined number of drainage openings.

20. The intermittent urinary catheter as in any of the preceding embodiments, provided with multiple drainage openings configured for providing a total inflow area exceeding an inside lumen in the catheter just distally of the most distal of the drainage openings.

21. The intermittent urinary catheter as in any of the preceding embodiments, wherein the largest dimension of an individual drainage opening in an outer convex surface of the tubular portion is less than 1 mm.

22. The intermittent urinary catheter as in any of the preceding embodiments, wherein each of the drainage openings has a cross-sectional area of less than 0.8 mm$^2$.

23. The intermittent urinary catheter as in any of the preceding embodiments, wherein the largest dimension of an individual drainage opening in an outer convex surface of the tubular portion is less than 0.7 mm.

24. The intermittent urinary catheter as in any of the preceding embodiments, wherein each of the drainage openings has a cross-sectional area of less than 0.4 mm$^2$.

25. The intermittent urinary catheter as in any of the preceding embodiments, wherein the largest dimension of an individual drainage opening in an outer convex surface of the tubular portion is less than 0.5 mm.

26. The intermittent urinary catheter as in any of the preceding embodiments, wherein each of the drainage openings has a cross-sectional area of less than 0.2 mm$^2$.

27. The intermittent urinary catheter as in any of the preceding embodiments, wherein the number of drainage openings is more than 20.

28. The intermittent urinary catheter as in any of the preceding embodiments, wherein the number of drainage openings is 24.

29. The intermittent urinary catheter as in any of the preceding embodiments, wherein the number of drainage openings is 48.

30. The intermittent urinary catheter as in any of the preceding embodiments, wherein the number of drainage openings is 56.

31. The intermittent urinary catheter as in any of the preceding embodiments, wherein the number of drainage openings is 68.

32. The intermittent urinary catheter as in any of the preceding embodiments, wherein the number of drainage openings is 108.

33. The intermittent urinary catheter as in any of the preceding embodiments, wherein the number of drainage openings is 144.

34. The intermittent urinary catheter as in any of the preceding embodiments, wherein the number of drainage openings is more than 50.

35. The intermittent urinary catheter as in any of the preceding embodiments, wherein the number of drainage openings is more than 100.

36. The intermittent urinary catheter as in any of the preceding embodiments, wherein the number of drainage openings is more than 200.

37. The intermittent urinary catheter as in any of the preceding embodiments, wherein the number of drainage openings is more than 250.

38. The intermittent urinary catheter as in any of the preceding embodiments, wherein the catheter is provided with more than 12 drainage openings and each drainage opening has a largest dimension in an outer convex surface of the tubular portion of approximately 0.8 mm.

39. The intermittent urinary catheter as in any of the preceding embodiments, wherein the catheter is provided with 48 drainage openings and each drainage opening has a largest dimension in an outer convex surface of the tubular portion of approximately 0.4 mm.

40. The intermittent urinary catheter as in any of the preceding embodiments, wherein the catheter is a CH10, each drainage opening has largest dimension in an outer convex surface of the tubular portion of approximately 0.4 mm, and the number of drainage openings is larger than 32.

41. The intermittent urinary catheter as in any of the preceding embodiments, wherein the catheter is a CH12, each drainage opening has largest dimension in an outer convex surface of the tubular portion of approximately 0.4 mm, and the number of drainage openings is larger than 44.

42. The intermittent urinary catheter as in any of the preceding embodiments, wherein the catheter is a CH14, each drainage opening has largest dimension in an outer convex surface of the tubular portion of approximately 0.4 mm, and the number of drainage openings is larger than 66.

43. The intermittent urinary catheter as in any of the preceding embodiments, wherein the catheter is a CH16, each drainage opening has largest dimension in an outer convex surface of the tubular portion of approximately 0.4 mm, and the number of drainage openings is larger than 88.

44. The intermittent urinary catheter as in any of the preceding embodiments, wherein the catheter is a CH12, each drainage opening has largest dimension in an outer convex surface of the tubular portion of approximately 0.7 mm, and the number of drainage openings is larger than 15.

45. The intermittent urinary catheter as in any of the preceding embodiments, wherein the catheter is a CH16, each drainage opening has largest dimension in an outer convex surface of the tubular portion of approximately 0.7 mm, and the number of drainage openings is larger than 29.

46. The intermittent urinary catheter as in any of the preceding embodiments, wherein each one of the drainage openings extends transversely to a longitudinal direction of the catheter.

47. The intermittent urinary catheter as in any of the preceding embodiments, wherein the closed tip portion has a length of less than 2 cm in a longitudinal direction of the catheter.

48. The intermittent urinary catheter as in any of the preceding embodiments, wherein the drainage portion has a length of 20 mm in a longitudinal direction of the catheter.

49. The intermittent urinary catheter as in any of the preceding embodiments, wherein the intermittent urinary catheter is a female catheter and the drainage portion has a length of approximately 25 mm in a longitudinal direction of the catheter.

50. The intermittent urinary catheter as in any of the preceding embodiments, wherein the drainage portion has a length of 4 cm in a longitudinal direction of the catheter.

51. The intermittent urinary catheter as in any of the preceding embodiments, wherein the drainage portion has a length of 10 cm in a longitudinal direction of the catheter.

52. The intermittent urinary catheter as in any of the preceding embodiments, wherein the drainage portion has a length of 15 cm in a longitudinal direction of the catheter.

53. The intermittent urinary catheter as in any of the preceding embodiments, wherein the intermittent urinary catheter is a male catheter and drainage portion has a length of approximately 8 cm in a longitudinal direction of the catheter.

54. The intermittent urinary catheter as in any of the preceding embodiments, wherein the intermittent urinary catheter is a female catheter and the drainage portion has a length of approximately 4 cm in a longitudinal direction of the catheter

55. The intermittent urinary catheter as in any of the preceding embodiments, wherein the drainage portion is divided into a first drainage portion a second drainage portion.

56. The intermittent urinary catheter as in any of the preceding embodiments, wherein the second drainage portion is positioned distally of the first drainage portion.

57. The intermittent urinary catheter as in any of the preceding embodiments, wherein the first drainage portion is configured for being positioned in the bladder during use.

58. The intermittent urinary catheter as in any of the preceding embodiments, wherein the second drainage portion is configured for being positioned towards the bottom of the bladder and in the upper part of the urethra during use.

59. The intermittent urinary catheter as in any of the preceding embodiments, wherein drainage openings are positioned scattered in the longitudinal direction as well as around the circumference of the catheter.

60. The intermittent urinary catheter as in any of the preceding embodiments, wherein the drainage openings are positioned in three longitudinal rows with 120 degrees between them around the circumference of the catheter.

61. The intermittent urinary catheter as in any of the preceding embodiments, wherein the drainage openings are positioned in four longitudinal rows with 90 degrees between them around the circumference of the catheter.

62. The intermittent urinary catheter as in any of the preceding embodiments, wherein the drainage openings are positioned in 6 longitudinal rows with 60 degrees between them around the circumference of the catheter.

63. The intermittent urinary catheter as in any of the preceding embodiments, wherein the drainage openings are positioned in 8 longitudinal rows with 45 degrees between them around the circumference of the catheter.

64. The intermittent urinary catheter as in any of the preceding embodiments, wherein the drainage openings are positioned in two longitudinal rows with 180 degrees between them around the circumference of the catheter.

65. The intermittent urinary catheter as in any of the preceding embodiments, wherein the drainage openings are positioned in two pairs of parallel rows with 180 degrees between the rows around the circumference of the catheter.

66. The intermittent urinary catheter as in any of the preceding embodiments, wherein the drainage openings are helically dispersed around the circumference of the catheter.

67. The intermittent urinary catheter as in any of the preceding embodiments, wherein the drag-force is less than 0.6 mN in a distance of 1 mm from the drainage opening, when a flow rate through the catheter is in the range of 2 ml/s to 10 ml/s.

68. The intermittent urinary catheter as in any of the preceding embodiments, wherein the drag-force is less than 0.2 mN in a distance of 1 mm from the drainage opening, when a flow rate through the catheter is in the range of 2 ml/s to 10 ml/s.

69. The intermittent urinary catheter of any of the preceding embodiments, wherein the drainage portion has a first degree of perforation of 0.4-0.6 in a proximal portion of the drainage portion and a second degree of perforation of 0.05-0.3 distally thereof.

70. The intermittent urinary catheter of any of the preceding embodiments, wherein the first drainage portion as a first degree of perforation and wherein the second drainage portion has a second degree of perforation.

71. The intermittent urinary catheter of any of the preceding embodiments, wherein the drainage portion has a first degree of perforation of 0.5-0.7, a second degree of perforation of 0.3-0.5 and a third degree of perforation of 0.05-0.3.

72. The intermittent urinary catheter of any of the preceding embodiments, wherein the drainage portion has a first degree of perforation of approximately 0.02 and a second degree of perforation of approximately 0.01

73. The intermittent urinary catheter of any of the preceding embodiments, wherein the threshold limit is 200mBar when tested as described herein.

74. The intermittent urinary catheter of any of the preceding embodiments, wherein the threshold limit is 100mB$\alpha$r when tested as described herein.

75. The intermittent urinary catheter of any of the preceding embodiments, wherein the pressure ratio is less than 70 at any point in a distance of 1 mm externally from the drainage portion.

76. The intermittent urinary catheter of any of the preceding embodiments, wherein the pressure ratio is less than 50 at any point in a distance of 1 mm externally from the drainage portion.

77. The intermittent urinary catheter of any of the preceding embodiments, wherein the pressure ratio is less than 20 at any point in a distance of 1 mm externally from the drainage portion.

78. The intermittent urinary catheter of any of the preceding embodiments, wherein the pressure ratio is less than 10 at any point in a distance of 1 mm externally from the drainage portion.

79. The intermittent urinary catheter of any of the preceding embodiments, wherein the pressure ratio is than 5 at any point in a distance of 1 mm externally from the drainage portion.

80. The intermittent urinary catheter of any of the preceding embodiments, wherein the factor between maximum inflow and minimal inflow is less than 50.

81. The intermittent urinary catheter of any of the preceding embodiments, wherein the factor between maximum inflow and minimal inflow is less than 20.

82. The intermittent urinary catheter of any of the preceding embodiments, wherein the factor between maximum inflow and minimal inflow is less than 10.

83. The intermittent urinary catheter of any of the preceding embodiments, wherein the factor between maximum inflow and minimal inflow is less than 5.

84. The intermittent urinary catheter of any of the preceding embodiments, wherein the ratio of the open drain area to the drainage area on the exterior surface of the tubular portion is in a range from 0.05 to 0.20.

85. The intermittent urinary catheter of any of the preceding embodiments, wherein the ratio of the open drain area to the drainage area on the exterior surface of the tubular portion is in a range from 0.2 to 0.5.

86. The intermittent urinary catheter of any of the preceding embodiments, wherein the ratio of the open drain area to the drainage area on the exterior surface of the tubular portion is in a range from 0.3 to 0.6.

87. The intermittent urinary catheter of any of the preceding embodiments, wherein the ratio of the open drain area to the drainage area on the exterior surface of the tubular portion is in a range from 0.4 to 0.7.

88. The intermittent urinary catheter of any of the preceding embodiments, wherein the open surface area comprises from 5% to 20% of the drainage area on the exterior surface of the tubular portion.

89. The intermittent urinary catheter of any of the preceding embodiments, wherein the open surface area comprises from 20% to 50% of the drainage area on the exterior surface of the tubular portion.

90. The intermittent urinary catheter of any of the preceding embodiments, wherein the open surface area comprises from 30% to 60% of the drainage area on the exterior surface of the tubular portion.

91. The intermittent urinary catheter of any of the preceding embodiments, wherein the open surface area comprises from 40% to 70% of the drainage area on the exterior surface of the tubular portion.

92. The intermittent urinary catheter as in any of the preceding embodiments, wherein the tip portion of the catheter is a Nelaton tip.

93. The intermittent urinary catheter as in any of the preceding embodiments, wherein the tip portion is a flex tip.

94. The intermittent urinary catheter as in any of the preceding embodiments, wherein the tip of the tip portion is closed.

95. A method of reducing the suction pressure fluctuation at the drainage openings in the bladder as a result of blocking of the drainage openings, by using an intermittent urinary catheter as in any of the preceding embodiments.

96. A method of reducing the pressure pulse to below predetermined threshold value, when tested as described herein, by using an intermittent urinary catheter as in any of the preceding embodiments.

97. The method of embodiment 96, wherein the predetermined threshold value is 350 mBar.

98. The method of embodiment 96, wherein the predetermined threshold value is 300 mBar.

99. A method of reducing a drag-force provided by the drainage openings in a distance of 1 mm from the drainage openings to a level below 1 mN during flow in the lumen below 10 ml/s.

100. A method of providing an intermittent urinary catheter that is configured for emptying without repositioning of the catheter during the draining process.

101. A method of emptying a bladder by using an intermittent urinary catheter as in any of the preceding embodiments, wherein the method does not require the step of repositioning of the catheter during the emptying procedure.

102. A method of emptying a bladder by using an intermittent urinary catheter as in any of the preceding embodiments, wherein emptying of the bladder is performed while the catheter is held stationary during the emptying procedure.

103. Use of an intermittent urinary catheter according to any of the preceding embodiments, wherein the use does not require the step of repositioning of the catheter during the emptying procedure.

104. Use of an intermittent urinary catheter according to any of the preceding embodiments, wherein the catheter is held stationary during the emptying procedure.

105. Use of an intermittent urinary catheter according to any of the preceding embodiments, wherein a flow-rate through the catheter exceeds zero ("0") through-out the catheterisation procedure.

106. Use of an intermittent urinary catheter according to any of the preceding embodiments, wherein the catheter is inserted into the urethra until the drainage portion reaches the bladder, the catheter is held in position during the draining of urine, and the catheter is removed, wherein the catheter is configured for draining the bladder without repositioning of the catheter during the draining of urine.

107. Use of an intermittent urinary catheter according to any of the preceding embodiments, wherein the catheter is inserted into the urethra until the drainage portion reaches the bladder, the catheter is held in position during the draining of urine, and the catheter is removed, wherein the catheter is configured for draining the bladder completely while the catheter is held stationary during the draining of urine.

108. Use of an intermittent urinary catheter according to any of embodiments 1 to 79, wherein the catheter is configured for reducing the drag-force provided by the drainage openings to a level below 1mN in a distance of 1 mm from the drainage openings, when the flow rate is less than 10 ml/s.

109. A method of draining urine from a bladder, the method comprising:

providing an intermittent urinary catheter having tubular portion extending between a tip portion forming a tip at a proximal insertion end and a distal outlet end, with the tubular portion having a lumen and drainage openings formed through the tubular portion communicating with the lumen;

distributing the drainage openings along a drainage portion of the tubular portion of the intermittent catheter;

configuring a total sum of a cross-sectional area of the drainage openings to be at least two times greater than a cross-sectional area of the lumen; and

instructing a user to insert the intermittent urinary catheter into a urethra until the tip portion of the intermittent urinary catheter is positioned inside of a bladder.

110. A method of draining urine from a bladder, the method comprising:

providing an intermittent urinary catheter having tubular portion extending between a tip portion forming a tip at a proximal insertion end and a distal outlet end, with the tubular portion having a lumen and drainage openings formed through the tubular portion communicating with the lumen;

distributing the drainage openings along a drainage portion of the tubular portion of the intermittent catheter;

instructing a user to insert the intermittent urinary catheter into a urethra until the tip portion of the intermittent urinary catheter is positioned inside of a bladder; and

configuring the tubular portion of the intermittent urinary catheter to have a suction pressure below 50 mBar measured inside the lumen during drainage of urine from the bladder.

**Claims**

1. An intermittent urinary catheter having a tubular portion extending from a proximal insertion end to

   a distal outlet end, with the tubular portion formed to include a lumen adapted to transport urine through the intermittent urinary catheter;
   a drainage area provided on an exterior surface of the tubular portion, where the drainage area includes a closed surface area and an open surface area, where the open surface area of the drainage area allows urine to enter the lumen;
   wherein the drainage area is defined by a length measured as a longitudinal distance from a proximal most edge of the open surface area to a distal most edge of the open surface area multiplied by a circumference of the tubular portion measured within the length of the drainage area;
   wherein the open surface area comprises from 5% to 70% of the drainage area on the exterior surface of the tubular portion.

2. The intermittent urinary catheter of any of the preceding claims, wherein the open surface area comprises from 5% to 20% of the drainage area on the exterior surface of the tubular portion.

3. The intermittent urinary catheter of any of the preceding claims, wherein the open surface area comprises from 20% to 50% of the drainage area on the exterior surface of the tubular portion.

4. The intermittent urinary catheter of any of the preceding claims, wherein the open surface area comprises from 30% to 60% of the drainage area on the exterior surface of the tubular portion.

5. The intermittent urinary catheter of any of the preceding claims, wherein the open surface area comprises from 40% to 70% of the drainage area on the exterior surface of the tubular portion.

6. The intermittent urinary catheter as in any of the preceding claims, wherein the open surface area comprises multiple drainage openings.

7. The intermittent urinary catheter as in any of the preceding claims, wherein the open surface area comprises more than 12 drainage openings or more than 20 drainage openings, such as 48, 56, 68, 108 or 144.

8. The intermittent urinary catheter as in any of the preceding claims, wherein the open surface area comprises multiple drainage openings positioned with a distance of 1.4 mm between them.

9. The intermittent urinary catheter as in any of the preceding claims, wherein the open surface area comprises multiple drainage openings positioned with a distance of 3.4 mm between them.

10. The intermittent urinary catheter as in any of the preceding claims, wherein the drainage openings are positioned in longitudinal rows.

11. The intermittent urinary catheter as in any of the preceding claims, wherein the largest dimension of an individual drainage opening in an outer convex surface of the tubular portion is less than 1 mm.

12. The intermittent urinary catheter as in any of the preceding claims, wherein the drainage openings comprise a closed loop circumference, such as circular, oval, square, triangular and any other closed loop shape.

13. The intermittent urinary catheter as in any of the preceding claims, wherein the longitudinal distance from a proximal most edge of the open surface area to a distal most edge of the open surface area is at least 20 mm.

14. The intermittent urinary catheter as in any of the preceding claims, wherein the open surface area is at least 4.8 mm$^2$.

15. The intermittent urinary catheter as in claim 13, wherein the longitudinal distance from a proximal most edge of the open surface area to a distal most edge of the open surface area is 25 mm, or 4 cm or 8 cm or 10 cm or 15 cm.

16. The intermittent urinary catheter as in any of the preceding claims, wherein the open surface area is at least 6 mm$^2$.

17. The intermittent urinary catheter as in any of the preceding claims, wherein the open surface area is at least 11 mm$^2$.

18. The intermittent urinary catheter as in any of the preceding claims, wherein the open surface area is less than 28.8 mm$^2$.

19. The intermittent urinary catheter as in any of the preceding claims, wherein the tubular portion comprises a drainage portion provided with drainage openings for allowing urine to enter the inside lumen.

20. The intermittent urinary catheter as claim 19, wherein the drainage portion is divided into a first drainage portion and a second drainage portion.

21. The intermittent urinary catheter as in claim 20, wherein the second drainage portion is positioned distally of the first drainage portion.

22. The intermittent urinary catheter as in any of claims 20 or 21, wherein the first drainage portion is configured for being positioned in the bladder during use.

23. The intermittent urinary catheter as in any of claims 20 to 22, wherein the second drainage portion is configured for being positioned towards the bottom of the bladder and in the upper part of the urethra during use.

24. The intermittent urinary catheter as in any of the preceding claims, wherein the drainage area comprises drainage openings configured for allowing urine to enter the inside lumen, a number and size of the of drainage openings being configured for providing a non-interrupted flow.

25. The intermittent urinary catheter as in any of the preceding claims, wherein the catheter is configured for being held in position during draining of urine from the bladder, such that the catheter is configured for draining the bladder without repositioning of the catheter during the draining of urine.

Fig. 1

Fig. 2

Fig. 3

Fig. 4

Fig. 5

Fig. 6

Fig. 7

Fig. 8

Fig. 9A

Fig. 9B

Fig. 10A

Fig. 10B

Fig. 11A

Fig. 11B

Fig. 12A

Fig. 12B

Fig. 13A

Fig. 13B

Fig. 14

Fig. 15A

Fig. 15B

Fig. 16

Fig. 17

Pressure Peak (mBar)

Fig. 18

Fig. 19

Fig. 20

EP 4 327 855 A2

Pressure Peak [mBar] vs. Diameter of Circle with same Area [mm]

Fig. 21A

Fig. 21B

Fig. 22

Fig. 23

Fig. 24

Fig. 25

Fig. 26

Fig. 27

Fig. 28

Fig. 29

Number of cloggings [%]

Fig. 30

Residual at first flowstop [g]

Fig. 31

Fig. 32

Fig. 33

Fig. 34

Fig. 35

Fig. 36

EP 4 327 855 A2

09/25/2019 14:18:43.66024236

Fig. 37A

Fig. 37B

Fig. 38

Fig. 39

Fig. 40

Fig. 41

Fig. 42

400

401

404

402

403

Fig. 43

401

405

Fig. 44

406

Fig. 45

Fig. 46

Fig. 47

Fig. 48

Fig. 49

Fig. 50

Fig. 51

Fig. 52

Fig. 53

Fig. 54

Fig. 55

Fig. 56

Fig. 57

Fig. 58

Fig. 59

Fig. 60

Fig. 61

Fig. 62

Fig. 63

EP 4 327 855 A2

Fig. 64

Line Graph: abs(p) (Pa)

Legend:
11: Time=10 s, flow=2 ml/s
32: Time=10 s, flow=10 ml/s

Fig. 65

Line Graph: abs(p) (Pa)

Fig. 66

Line Graph: abs(p) (Pa)

Fig. 67

Fig. 68

Fig. 69

Fig. 70

Fig. 71

Fig. 72

Fig. 73

Fig. 74

Line Graph: abs(p) (Pa)

— 11: Time=10 s, flow=1 ml/s
— 22: Time=10 s, flow=5 ml/s

abs(p) (Pa)

Fig. 75

Fig. 76

Fig. 77